# EUROPEAN PATENT APPLICATION

(11) **EP 4 180 442 A1**
(43) Date of publication of application: **17.05.2023**
(21) Application number: 21836863.7
(22) Date of filing: 08.07.2021
(51) Int. Cl.: C07H 21/02, C07F 9/09

(54) **METHOD FOR PRODUCING NUCLEIC ACID OLIGOMER**

(30) Priority: 09.07.2020 JP 2020118316
(71) Applicant: SUMITOMO CHEMICAL COMPANY, LIMITED, Tokyo 103-6020 (JP)
(72) Inventor: TANAKA, Yuki, Osaka-shi, Osaka 555-0021 (JP); OKUMURA, Hideki, Osaka-shi, Osaka 555-0021 (JP); KANO, Toshifumi, Osaka-shi, Osaka 555-0021 (JP)
(74) Representative: Marks & Clerk LLP
(86) International application number: PCT/JP2021/025835
(87) International publication number: WO 2022/009959

(57) **Abstract**

An object of the present invention is to provide an efficient method for producing a nucleic acid oligomer. An object of the present invention is to provide a method for producing a nucleic acid oligomer represented by formula (2) (in the formula, the symbols have the meanings described in the specification), including a step of reacting a nucleic acid oligomer represented by formula (1) (in the formula, the symbols have the meanings described in the specification) with a trichloroacetic acid solution in which a molar ratio of formaldehyde to trichloroacetic acid (formaldehyde mol/trichloroacetic acid mol) is 11 × 10⁻⁵ or less.

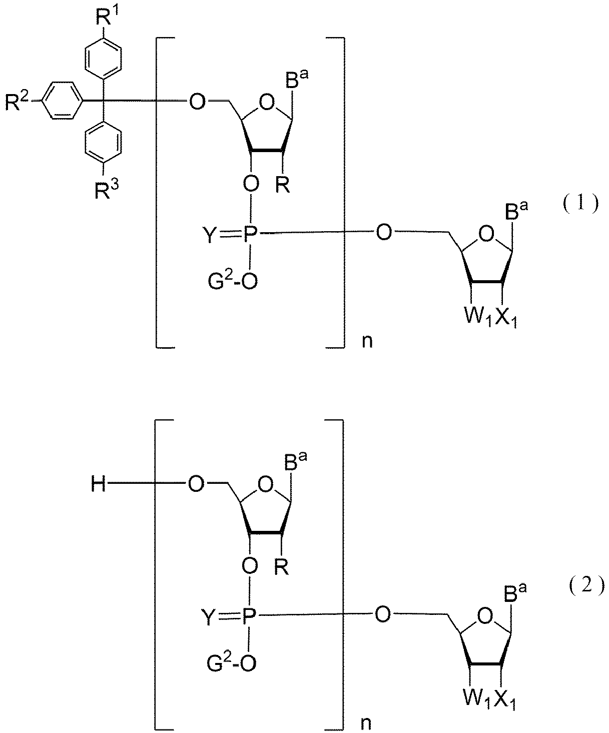

## Description

### TECHNICAL FIELD

This patent application claims the priority and benefit from the Paris Convention, based on Japanese Patent Application No. 2020-118316 (filed on July 9, 2020), the entire contents of which are incorporated herein by reference.

The present invention relates to a method for producing a nucleic acid oligomer.

### BACKGROUND ART

In recent years, the application of nucleic acid oligomers to the medical field has attracted increasing interest. Examples of the nucleic acid oligomers include antisense nucleic acids, aptamers, ribozymes, and nucleic acids which induce RNA interference (RNAi) such as siRNAs, which are referred to as nucleic acid therapeutics.

Nucleic acid oligomers can be synthesized by a solid-phase synthesis method, nucleic acid oligomers synthesized by elongating nucleic acids on solid supports are cut out from the solid supports, and for the nucleic acid oligomers including a ribose, a protecting group for a hydroxyl group at the 2'-position of the ribose is removed by deprotection to produce target nucleic acid oligomers. In the solid-phase synthesis method, a phosphoramidite (hereinafter, referred to as an "amidite") of a nucleoside is known to be used as a raw material, and a protecting group for a hydroxyl group at the 5'-position is known to be deprotected with the use of a trichloroacetic acid solution, but the yield of a nucleic acid oligomer synthesized with the use of a conventional trichloroacetic acid solution has not necessarily been satisfactory, thereby resulting an insufficient synthesis (Patent Document 1).

### PRIOR ART DOCUMENT

### PATENT DOCUMENT

Patent Document 1: WO-A-2006/022323

### SUMMARY OF THE INVENTION

### PROBLEMS TO BE SOLVED BY THE INVENTION

An object of the present invention is to provide an efficient method for producing a nucleic acid oligomer.

### MEANS FOR SOLVING THE PROBLEMS

As a result of intensive studies conducted by the inventors to achieve the object mentioned above, the inventors provide a method for efficiently producing a nucleic acid oligomer, characterized by using, in the synthesis of a nucleic acid oligomer, a trichloroacetic acid solution that is equal to or less than a certain level in formaldehyde concentration, or a trichloroacetic acid solution after improving the quality of the trichloroacetic acid.

The present invention encompasses, but is not to be considered limited to, the following aspects.

Item 1. A method for producing a nucleic acid oligomer represented by formula (2), the method comprising a step of reacting a nucleic acid oligomer represented by formula (1): 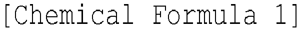 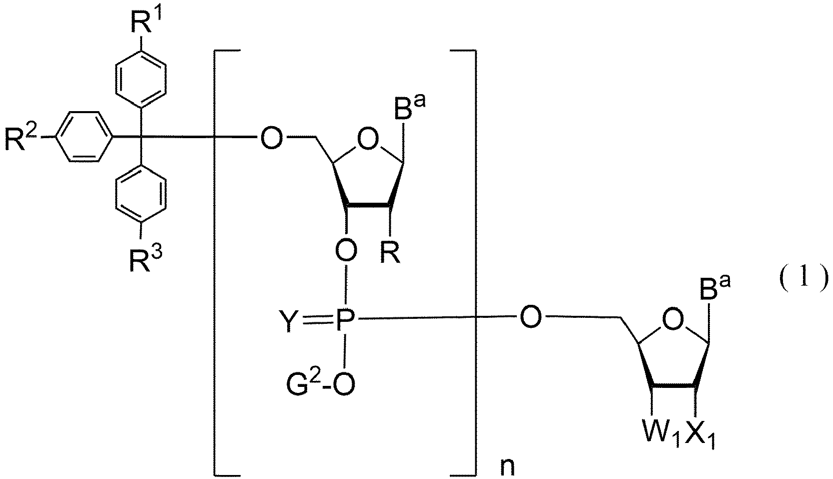 (in the formula,
G² represents a protecting group for a hydroxyl group,
B^{a} is the same or different and each independently represents a nucleobase optionally protected with a protecting group,
R¹, R², and R³ are the same or different and each independently represent a hydrogen atom or an alkoxy group,
R is the same or different and each independently represents a protected hydroxyl group, a hydrogen atom, a fluorine atom, a methoxy group, a 2-methoxyethyl group, or an OQ' group,
Q' is the same or different and each independently represents a methylene group bonded to a carbon atom at a 4'-position of a ribose, an ethylene group bonded to a carbon atom at a 4'-position of a ribose, or an ethylidene group bonded to a carbon atom at a 4'-position of a ribose,
Y is the same or different and each independently represents an oxygen atom or a sulfur atom,
n represents any integer of 1 to 200,
W₁ represents an OZ group, and X₁ represents an R group, or
W₁ represents an OV group, and X₁ represents an OZ group,
V represents a protecting group for a hydroxyl group, and
Z is a group that has a structure including a solid support and a linking group, and
when n is an integer of 2 or more, the nucleic acid oligomer represented by the formula (1) may have a non-nucleotide linker incorporated between respective nucleotides)
with a trichloroacetic acid solution in which the molar ratio of formaldehyde to trichloroacetic acid (formaldehyde mol/trichloroacetic acid mol) is 11 × 10⁻⁵ or less, 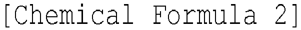 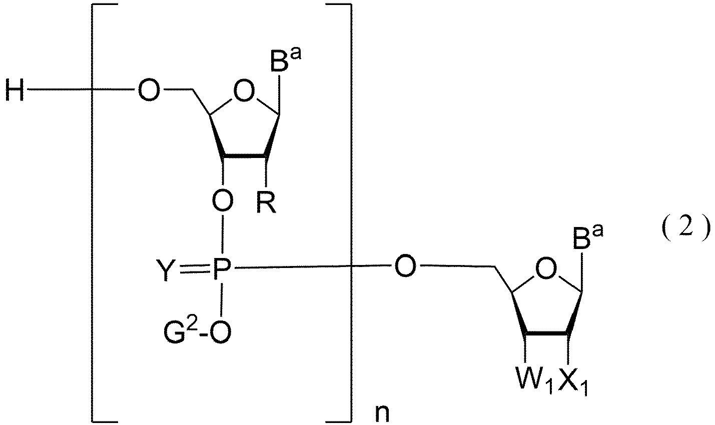 (in the formula,
   G², B^{a}, R, Y, X₁, W₁, and n are as described above, and
   a non-nucleotide linker is optionally incorporated between the nucleotides as defined in the formula (1)).

Item 2. A method for producing a nucleic acid oligomer represented by formula (2'), the method comprising: the step described in the item 1; and further a step of removing a group represented by Z from the nucleic acid oligomer represented by the formula (2), produced in the step described in the item 1; and a step of removing protecting groups for a hydroxyl group and a nucleobase, 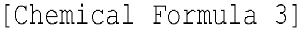 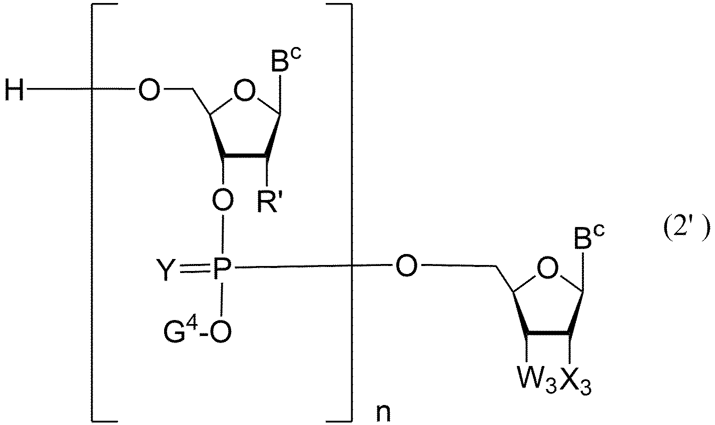 (in the formula,
Y and n are as described above,
B^{c} is the same or different and each independently represents a nucleobase,
G⁴ is the same or different and each independently represents a hydrogen atom, an alkali metal ion, an ammonium ion, an alkylammonium ion, or a hydroxyalkylammonium ion,
R' is the same or different and each independently represents a hydroxyl group, a hydrogen atom, a fluorine atom, a methoxy group, a 2-methoxyethyl group, or an OQ' group,
Q' is as described above, and
X₃ and W₃ each independently represent a hydroxyl group, or
X₃ represents an R' group, and W₃ represents a hydroxyl group).

Item 3. The production method according to the item 1, further comprising:
a step of optionally elongating a chain length of the nucleic acid oligomer represented by the formula (2) by an amidite method to obtain a nucleic acid compound represented by formula (3): 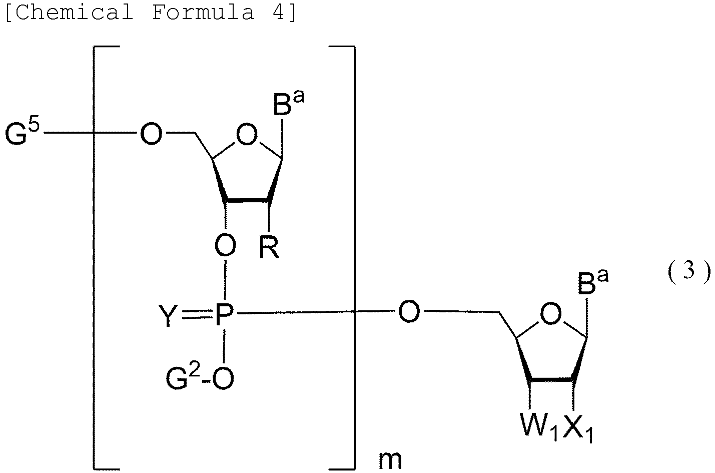 (in the formula,
G², B^{a}, R, Y, X₁, and W₁ are as described above,
G⁵ represents a protecting group for a hydroxyl group, represented by formula: 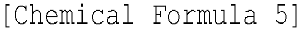 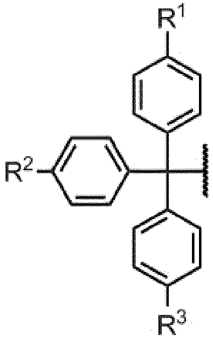
or a hydrogen atom,
R¹, R², and R³ are as described above, and
m is an integer that satisfies m ≥ n);
a step of cutting out, from the compound represented by the formula (3), a compound represented by formula (4): 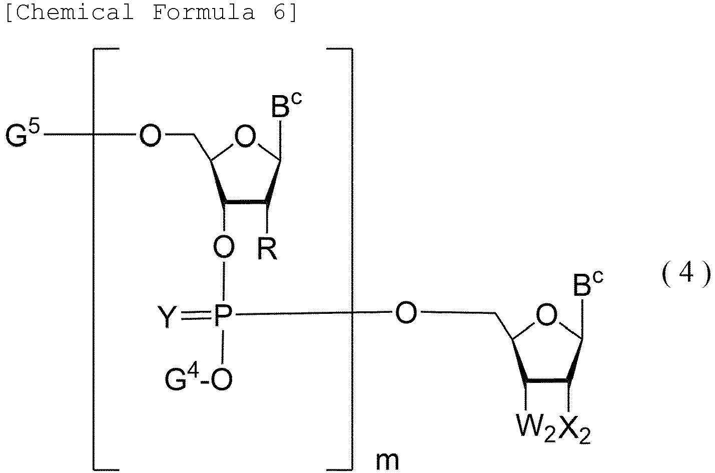 (in the formula,
   G⁵, R, Y, and m are as described above,
   G⁴ is the same or different and each independently represents a hydrogen atom, an alkali metal ion, an ammonium ion, an alkylammonium ion, or a hydroxyalkylammonium ion,
   B^{C} is the same or different and each independently represents a nucleobase,
   X₂ represents a hydroxyl group, and W₂ represents an OV group, or
   X₂ represents an R group, and W₂ represents a hydroxyl group, and
   V represents a protecting group for a hydroxyl group); and further
   deprotecting the compound represented by the formula (4) to produce a nucleic acid oligomer represented by formula (5): 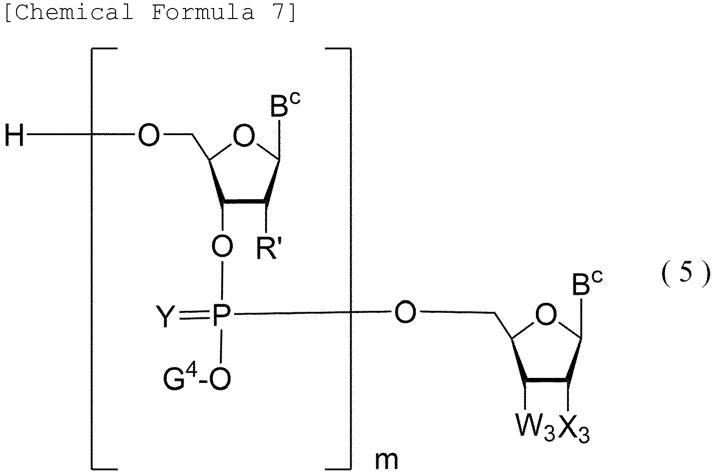 (in the formula,
      G⁴, B^{c}, Y, and m are as described above, and R' is the same or different and each independently represents a hydroxyl group, a hydrogen atom, a fluorine atom, a methoxy group, a 2-methoxyethyl group, or an OQ' group,
      Q' is as described above, and
      X₃ and W₃ each independently represent a hydroxyl group, or
      X₃ represents an R' group, and W₃ represents a hydroxyl group).

Item 4. The production method according to any one of the items 1 to 3, wherein the non-nucleotide linker is a linker comprising an amino acid skeleton.

Item 5. The production method according to the item 4, wherein the linker comprising the amino acid skeleton is a linker that has a structure selected from the group consisting of formulas (A14-1), (A14-2), and (A14-3) below: 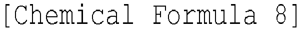 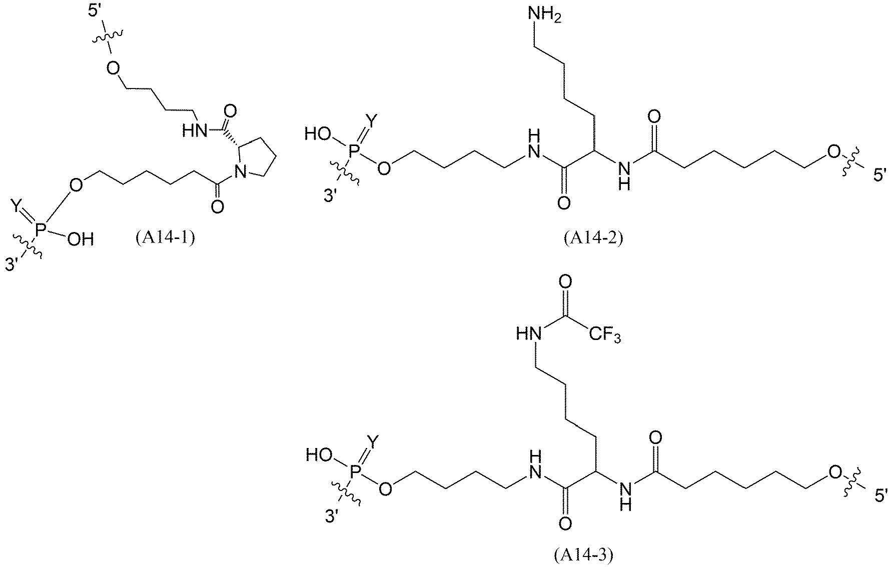 (in the formula, Y is as described above).

Item 6. The production method according to any one of the items 1 to 5, wherein the trichloroacetic acid solution comprises at least one solvent selected from the group consisting of dichloromethane, acetonitrile, and an aromatic organic solvent.

Item 7. The production method according to any one of the items 1 to 6, wherein the molar ratio of the formaldehyde to the trichloroacetic acid in the trichloroacetic acid solution (formaldehyde mol/trichloroacetic acid mol) is 54 × 10⁻⁶ or less.

Item 8. The production method according to any one of the items 1 to 6, wherein the molar ratio of the formaldehyde to the trichloroacetic acid in the trichloroacetic acid solution (formaldehyde mol/trichloroacetic acid mol) is 27 × 10⁻⁶ or less.

Item 9. The production method according to any one of the items 1 to 8, wherein the nucleic acid oligomer is ribonucleic acid (RNA).

Item 10. The production method according to any one of the items 1 to 8, wherein the nucleic acid oligomer is ribonucleic acid (RNA), and a protecting group for a hydroxyl group at a 2'-position of a ribose of the ribonucleic acid is a protecting group represented by formula (6), 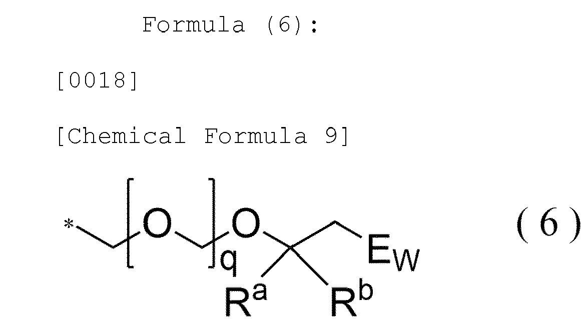 (in the formula,
q represents any integer of 1 to 5,
R^{a} and R^{b} are the same or different and each independently represent a methyl group, an ethyl group, or a hydrogen atom,
mark * represents a site bonded to an oxygen atom derived from the hydroxyl group at the 2'-position of the ribose, and
E_{W} represents an electron-withdrawing group).

Item 11. The production method according to the item 10, wherein R^{a} and R^{b} are simultaneously hydrogen atoms, and E_{W} is a cyano group.

Item 12. The production method according to any one of the items 1 to 11, wherein the nucleic acid oligomer is an oligomer with a chain length of 40 or more.

Item 13. The production method according to any one of the items 1 to 11, wherein the nucleic acid oligomer is an oligomer with a chain length of 50 or more.

Item 14. The production method according to any one of the items 1 to 11, wherein the nucleic acid oligomer is an oligomer with a chain length of 60 or more.

Item 15. The production method according to any one of the items 1 to 11, wherein the nucleic acid oligomer is an oligomer with a chain length of 80 or more.

Item 16. The production method according to any one of the items 1 to 11, wherein the nucleic acid oligomer is an oligomer with a chain length of 100 or more.

Item 17. A trichloroacetic acid solution, wherein a molar ratio of formaldehyde to trichloroacetic acid (formaldehyde mol/trichloroacetic acid mol) is 11 × 10⁻⁵ or less.

Item 18. The trichloroacetic acid solution according to the item 17, wherein the molar ratio of the formaldehyde to the trichloroacetic acid (formaldehyde mol/trichloroacetic acid mol) is 54 × 10⁻⁶ or less.

Item 19. The trichloroacetic acid solution according to the item 17, wherein the molar ratio of the formaldehyde to the trichloroacetic acid (formaldehyde mol/trichloroacetic acid mol) is 27 × 10⁻⁶ or less.

Item 20. A method for producing the trichloroacetic acid according to any one of the items 17 to 19, the method comprising a step of obtaining a purified trichloroacetic acid by azeotropically distilling away formaldehyde from a solution comprising: an unpurified trichloroacetic acid containing the formaldehyde; and a solvent for azeotropically distilling the formaldehyde.

Item 21. The production method according to the item 20, wherein the azeotropic solvent has a boiling point of 198°C or lower.

Item 22. The production method according to the item 20 or 21, wherein the azeotropic solvent is dichloromethane, acetonitrile, or an aromatic organic solvent.

Item 23. The production method according to the item 22, wherein the aromatic organic solvent is a toluene.

Item 24. A method for producing a nucleic acid oligomer, the method comprising: a step of purifying the trichloroacetic acid according to the item 20; and the step according to any one of the items 1 to 3, wherein the purified trichloroacetic acid obtained in the purifying step is used.

Item 25. The production method according to any one of the items 1 to 16 and 20 to 24, comprising a step of selecting, as a trichloroacetic acid solution, the trichloroacetic acid solution according to any one of the items 17, 18, and 19.

### EFFECT OF THE INVENTION

The present invention provides an efficient method for producing a nucleic acid oligomer. The yield of the nucleic acid oligomer to be produced can be expected to be improved by the production method according to the present invention.

### BRIEF DESCRIPTION OF THE DRAWING

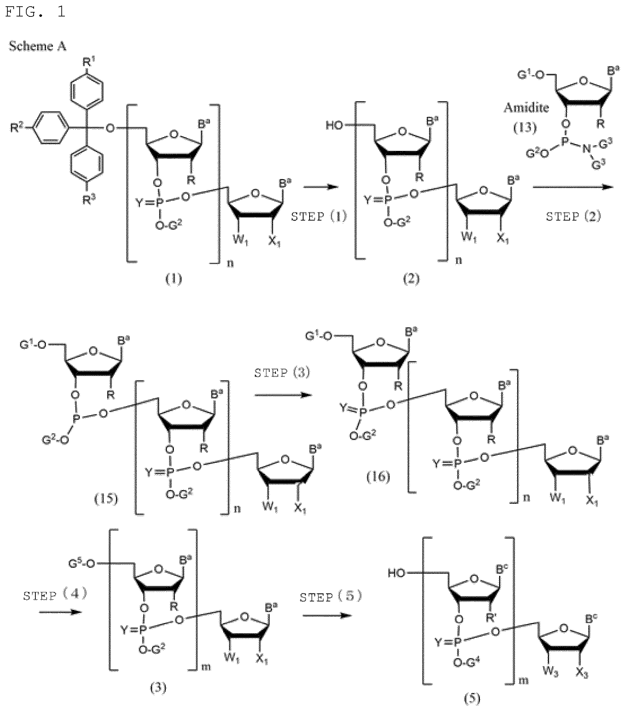

Fig. 1 is a Scheme A showing a typical example of producing a nucleic acid oligomer represented by the formula (5) from a nucleic acid oligomer represented by the formula (1). In the figure, any group can be used as G¹ without any particular limitation as long as the group can function as a protecting group for a hydroxyl group, and known protecting groups used in amidite compounds can be widely used. In addition, G³ is the same or different and each independently represents an alkyl groups, or two G³s may be bonded to each other to form a cyclic structure. Preferably, G³ is the same or different and each independently represents an alkyl group, for example, a methyl group, an ethyl group, a propyl group, or an isopropyl group, and both G³s are more preferably isopropyl groups. The other symbols are as described above.

### MODE FOR CARRYING OUT THE INVENTION

A method will be described in which a trichloroacetic acid solution that is equal to or less than a certain level in formaldehyde concentration is reacted with the nucleic acid oligomer represented by the formula (1) to obtain the nucleic acid oligomer represented by the formula (2).

The molar ratio of formaldehyde to trichloroacetic acid (formaldehyde mol/trichloroacetic acid mol) in the trichloroacetic acid solution according to the present invention is typically 11 × 10⁻⁵ or less, preferably 54 × 10⁻⁶ or less, and more preferably 27 × 10⁻⁶ or less. Examples of the trichloroacetic acid solution according to the present invention include a solution containing formaldehyde within the range of the molar ratio of the formaldehyde to the trichloroacetic acid. Examples of the method of measuring the formaldehyde concentration in the trichloroacetic acid solution include a high-performance liquid chromatographic method. In the high-performance liquid chromatographic method, formaldehyde and acetylacetone are reacted, the amount of 3,5-diacetyl-1,4-dihydrolutidine obtained is measured, and the concentration of the formaldehyde is calculated. In such a manner, a solution in the range of the molar ratio of the formaldehyde to the trichloroacetic acid can be selected. The solution may be directly prepared by, for example, the following azeotropic method, or a desired solution may be selected.

The concentration of the trichloroacetic acid in the trichloroacetic acid solution is typically 0.1 to 1.2 M, preferably 0.1 to 0.6 M, and more preferably 0.1 to 0.3 M.

The diluent solvent for the trichloroacetic acid is not particularly limited as long as the solvent is not involved in the reaction, and examples of the solvent can include dichloromethane, acetonitrile, an aromatic organic solvent, water, or any mixed solvent, preferably include at least one solvent selected from the group consisting of dichloromethane, acetonitrile, and an aromatic organic solvent, and more preferably include an aromatic organic solvent. Examples of the aromatic organic solvent include a toluene.

The reaction temperature in the reaction described above is preferably 0 to 40°C, and more preferably 10 to 30°C.

The formaldehyde in the trichloroacetic acid solution can be removed by azeotropy with any solvent or any mixed solvent, thereby reducing the amount thereof, and the azeotropic solvent is not particularly limited as long as the solvent is lower in boiling point lower than the trichloroacetic acid, and examples of the solvent can include dichloromethane, acetonitrile, an aromatic organic solvent, or any mixed solvent, preferably include dichloromethane, acetonitrile, or an aromatic organic solvent, and more preferably include an aromatic organic solvent. Examples of the aromatic organic solvent include a toluene.

The boiling point of the azeotropic solvent is preferably 200°C or lower, and more preferably 198°C or lower.

For the storage of the trichloroacetic acid solution, a glass container, a plastic container, or a metallic container can be used. As the plastic container, a container made of a polyethylene, a polypropylene or the like can be used, and as the metallic container, a container made of SUS, Hastelloy or the like can be used.

The oxidizing solution can be stored under an air atmosphere or an inert gas atmosphere, and as the inert gas, argon, nitrogen, carbon dioxide, helium, or the like can be used.

Examples of the nucleic acid compound having a protecting group for the hydroxyl group at the 5'-position include the nucleic acid compound represented by the formula (1). Examples of the nucleic acid compound produced by reacting the trichloroacetic acid solution include the nucleic acid compound represented by the formula (2).

Specific examples of the compound that independently represents an identical or different methylene group bonded to a carbon atom at the 4'-position of a ribose, an ethylene group bonded to a carbon atom at the 4'-position of a ribose, or an ethylidene group bonded to a carbon atom at the 4'-position of a ribose, represented by each Q', in the formulas (1) and (2) include structures represented by LNA-1, LNA-2, or LNA-3 in the following formula (7). 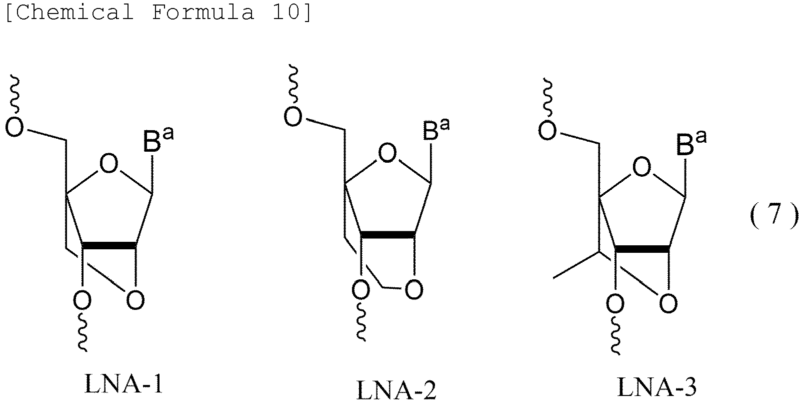 (in the formula, B^{a} represents an optionally protected nucleobase).

More specific examples of the group that has a structure including the solid support and the linking group connecting the solid support and the oxygen atom of a hydroxyl group at the 2'-position or 3'-position of a ribose at the 3'-terminal of the nucleic acid oligomer, represented by Z, include a structure represented by the following formula (8).

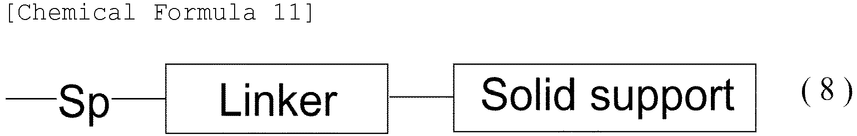

In the formula (8), Sp represents a spacer.

Examples of the spacer (Sp) include a spacer that has a structural formula represented by the following formula (9) .

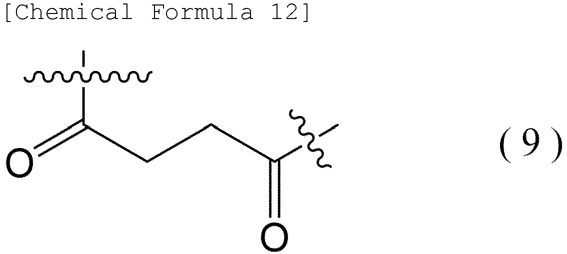

The Linker may have, for example, a structure represented by the following formula (10), or may have a structure represented by the formula (10), without any hexamethylene amino group moiety and with an aminopropyl group bonded to Si. Alternatively, Linker may have a structure represented by the following formula (11). 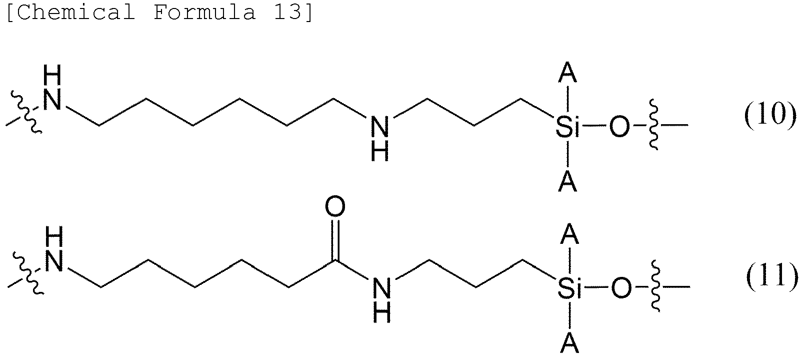 (in the formula,

A may be any of a hydroxyl group, an alkoxy group, and an alkyl group. Examples of the alkoxy group include a methoxy group and an ethoxy group. Examples of the alkyl group include a methyl group, an ethyl group, an isopropyl group, and a n-propyl group. Si indicates that Si is bonded to oxygen of a hydroxyl group on the carrier surface).

Examples of the solid support include an inorganic porous support and an organic resin support. Examples of the inorganic porous support include Controlled Pore Glass (CPG). Examples of the organic resin support include a support made of a polystyrene.

Examples of the nucleoside (ribose and deoxyribose) included in the nucleic acid oligomer for use in the present invention include DNA, RNA, 2'-O-MOE (2'-O-methoxyethyl), 2'-O-Me, 2'-F RNA, and LNA, but the nucleoside is not limited thereto.

The method for synthesizing a nucleic acid oligomer by a solid-phase synthesis method, including a deprotecting step with the trichloroacetic acid solution, typically includes the following steps:
(1) a step of deprotecting a hydroxyl group at the 5'-position of a nucleoside with a protected hydroxyl group bonded to a solid support with a linker interposed therebetween;
(2) a step of subjecting the hydroxyl group at the 5'-position, produced in the previous step, to a coupling reaction with a phosphoramidite compound to obtain a phosphite triester compound;
(3) a step of producing a nucleic acid molecule elongated by oxidizing the phosphite triester produced in the previous step and converting the phosphite triester into a phosphate triester, or an optional step of converting the phosphite triester into a thiophosphate triester;
(4) a step of synthesizing a nucleic acid molecule on the solid support by repeating, any number of times, a series of reaction cycles composed of the steps (1) to (3), that is, the step of deprotecting the hydroxyl group at the 5'-position of the produced nucleic acid molecule, the step of coupling the hydroxyl group at the 5'-position with the amidite compound, and the step of oxidizing the produced phosphite triester; and
(5) a step of subjecting the nucleic acid molecule on the solid support, produced in the step (4), to a step of cutting out and deprotecting the nucleic acid molecule, and releasing the nucleic acid molecule from the solid support to produce a nucleic acid oligomer with a protecting group removed therefrom.

The method for synthesizing a nucleic acid oligomer may, however, include, following the step (2) or (3), a step of capping the hydroxyl group at the 5'-position where the coupling reaction with the phosphoramidite compound fails to proceed, or the capping step may be added between any steps of the series of reaction cycles constituting the step (4).

The step (5) is more specifically performed by subjecting the nucleic acid molecule on the solid support, produced in the step (4), to the reactions of the following steps (5-1) and (5-2) in this order, and then subjecting the nucleic acid molecule to the reaction of the step (5-3). In this regard, the reaction of the step (5-1) may be developed optionally, and the reaction of the step (5-2) may be performed with the use of the method described in JP-B-4705716. As a result, a nucleic acid oligomer with a protecting group removed from a nucleic acid molecule released from the solid support or a nucleic acid oligomer with a protected hydroxyl group at the 5'-terminal can be produced.
(5-1) a reaction of deprotecting a protecting group for the hydroxyl group at the 5'-terminal of the nucleic acid molecule;
(5-2) a reaction of cutting out and releasing the nucleic acid molecule from the solid support; and
(5-3) a reaction of deprotecting a protecting group for a hydroxyl group at the 2'-position of a ribose constituting the nucleic acid molecule or the 3'-position of the 3'-terminal thereof.

The scheme of the steps (1) to (5) is shown in Fig. 1. The deprotecting reaction in the step (1) or step (4) shown in Fig. 1 is developed with the use of the trichloroacetic acid solution. The definitions of the substituents in the chemical formulas in the Scheme A are as defined above.

The nucleic acid compound of the formula (1) can be further elongated by any chain length with the use of a nucleotide-type or non-nucleotide-type linker in accordance with an amidite method, and used for the production of the nucleic acid compound represented by the formula (3). The nucleic acid oligomer represented by the formula (5) can be also obtained by cutting out only the nucleic acid compound from the nucleic acid compound bonded to the solid support, of the formula (3), to obtain the nucleic acid oligomer represented by the formula (4), and then further deprotecting the nucleic acid oligomer. Hereinafter, the substituents in each formula will be described in more detail.

The nucleobase optionally protected by the protecting group, represented by B^{a}, and the nucleobase represented by B^{c} are not particularly limited. Examples of the nucleobases include an adenine, a cytosine, a guanine, an uracil, a thymine, a 5-methylcytosine, a pseudouracil, and a 1-methylpseudouracil. In addition, the nucleobases may be substituted with a substituent. Examples of such a substituent include a halogen atom such as a fluoro group, a chloro group, a bromo group, or an iodo group, an acyl group such as an acetyl group, an alkyl group such as a methyl group or an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, and a nitro group, and combinations of two or more of these substituents.

The protecting group for the nucleobase, represented by B^{a}, and optionally protected with a protecting group, is not particularly limited, protecting groups for use in known nucleic acid chemistry can be used, and examples of such protecting groups include a benzoyl group, a 4-methoxybenzoyl group, a 4-methylbenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, and a (dimethylamino)methylene group, and combinations of two or more of these groups.

B^{a} more specifically represents a group represented by any of the following: 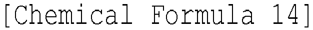 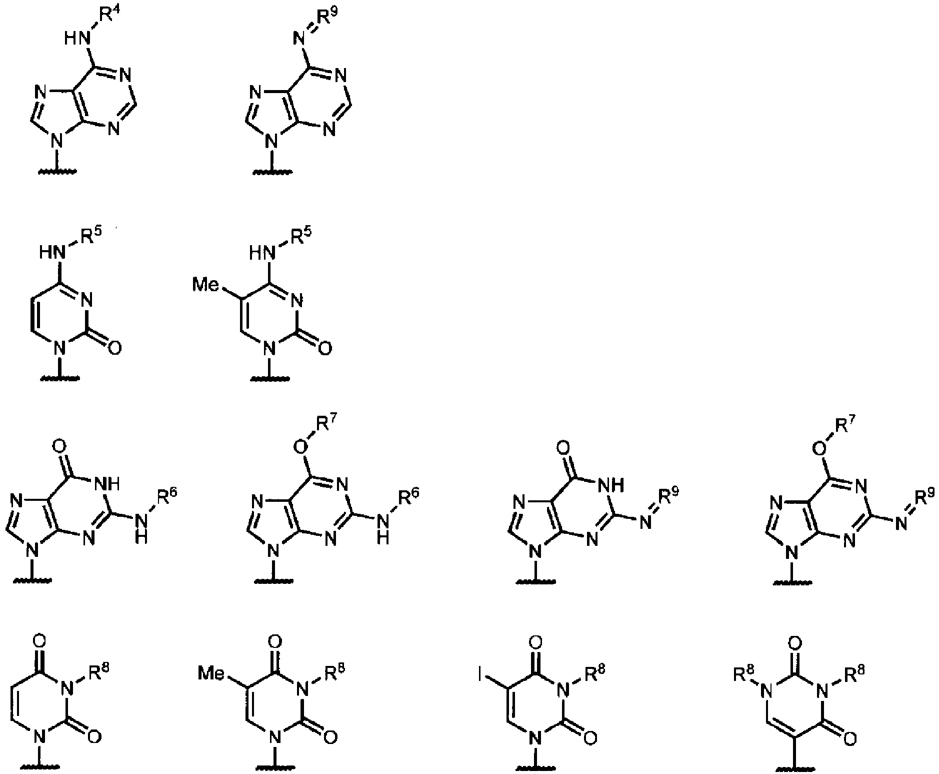 (in the formula described above,
R⁴ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
R⁵ represents a hydrogen atom, an acetyl group, an isobutyryl group, or a benzoyl group,
R⁶ represents a hydrogen atom, a phenoxyacetyl group, a 4-tert-butylphenoxyacetyl group, a 4-isopropylphenoxyacetyl group, a phenylacetyl group, an acetyl group, or an isobutyryl group,
R⁷ represents a 2-cyanoethyl group,
R⁸ represents a hydrogen atom, a methyl group, a benzoyl group, a 4-methoxybenzoyl group, or a 4-methylbenzoyl group, and
R⁹ represents a dimethylaminomethylene group).

More specific examples of B^{c} include groups obtained by removing the protecting groups from the above-described specific examples of B^{a}.

G⁵ is preferably the following group. 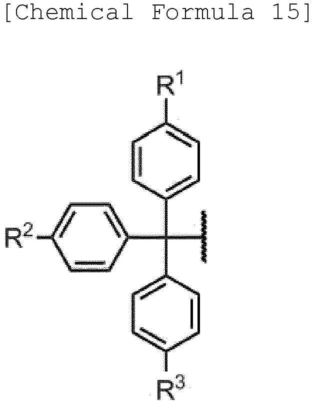 (in the formula,
R¹, R², and R³ are the same or different and each independently represent a hydrogen atom or an alkoxy group) .

Preferably, one of R¹, R², and R³ is a hydrogen atom, whereas the remaining two thereof are alkoxy groups which are the same or different (preferably the same), and the alkoxy groups are particularly preferably methoxy groups. More preferably, G⁵ is a 4,4'-dimethoxytrityl group (DMTr group) .

Any group can be used as G² without any particular limitation as long as the group can function as a protecting group for a hydroxyl group, and known protecting groups used in amidite compounds can be widely used. Examples of G² include an alkyl group, an alkenyl group, an alkynyl group, a cycloalkyl group, a haloalkyl group, an aryl group, a heteroaryl group, an arylalkyl group, a cycloalkenyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono-, di-, or trialkylsilyl group, and a mono-, di-, or trialkylsilyloxyalkyl group, and these groups are optionally substituted with one or more electron-withdrawing groups.

G² is preferably an alkyl group substituted with an electron-withdrawing group. Examples of the electron-withdrawing group include a cyano group, a nitro group, an alkylsulfonyl group, a halogen atom, an arylsulfonyl group, a trihalomethyl group, and a trialkylamino group, and a cyano group is preferred.

Particularly preferred as G² are the following group.

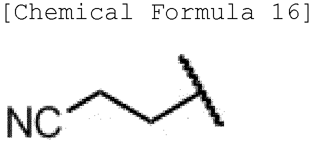

The alkyl groups in the definitions of R¹, R², R³, and G² may be linear or branched, and is preferably an alkyl group having 1 to 12 carbon atoms, and more preferably an alkyl group having 1 to 6 carbon atoms. Specific examples of the alkyl groups include a methyl group, an ethyl group, a n-propyl group, an isopropyl group, a n-butyl group, an isobutyl group, a tert-butyl group, a n-pentyl group, an isopentyl group, and a hexyl group. The alkyl group moiety constituting the alkoxy group in the definition of the substituent has the same definition as the definition of the alkyl group herein.

In addition, in the method according to the present invention, the amidite compound can be used in a free state or a salt state. Examples of the salt of the amidite compound include, but not be particularly limited to, a base addition salt or an acid addition salt. Specific examples of the base addition salt include salts with inorganic bases, such as a sodium salt, a magnesium salt, a potassium salt, a calcium salt, and an aluminum salt; salts with organic bases, such as a methylamine, an ethylamine, and an ethanolamine; salts with basic amino acids, such as a lysine, an ornithine, and an arginine; and ammonium salts. Specific examples of the acid addition salt include mineral acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethanesulfonic acid, and ethanesulfonic acid; and acid addition salts with acidic amino acids such as aspartic acid and glutamic acid. The amidite compound also encompasses forms such as salts, hydrates, solvates, and crystal polymorphs.

R preferably represents a protected hydroxyl group. The protecting group in the case of R representing a protected hydroxyl group or the protecting group of the hydroxyl group represented by V may be any group that can be used in the amidite method, and for example, the groups described in WO-A-2013/027843 and WO-A-2019/208571 can be used in addition to a 2'-tert-butyldimethylsilyl (TBS) group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group, a 2'-para-toluylsulfonylethoxymethyl (TEM) group, and a 2'-EMM group (WO-A-2006/022323). V is preferably a 2'-tert-butyldimethylsilyl (TBS) group. In addition, when the nucleic acid oligomer includes therein a ribose, for example, when the nucleic acid oligomer produced by the method according to the present invention is ribonucleic acid (RNA), as a protecting group for the hydroxyl group at the 2'-position of the ribose, the protecting group represented by the formula (6) is exemplified as a preferred protecting group. More preferably, a protecting group represented by formula (12) having a cyano group is exemplified as an electron-withdrawing group represented by E_{W}. 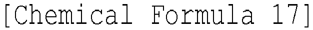 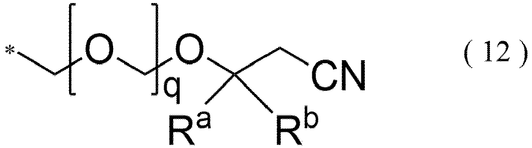 (in the formula,
q, R^{a}, and R^{b} have the same meaning as defined in the formula (6)).

More preferably, a group where R^{a} and R^{b} are simultaneously hydrogen atoms with q of 1 is exemplified for the group represented by the formula (12).

The protecting group represented by the formula (12) can be synthesized, for example, in accordance with the description of WO-A-2013/027843 and WO-A-2019/208571, and an amidite compound having such a protecting group can be used for the production of a nucleic acid compound.

The amidite compound of the formula (13) listed in the Scheme A of Fig. 1 is used for the elongation reaction of the nucleic acid.

Examples of the non-nucleotide linker include a linker including an amino acid skeleton (for example, a linker including the amino acid skeleton described in JP-B-5157168 or JP-B-5554881). Specifically, non-limiting examples of the non-nucleotide linker include a linker represented by the formula (A14-1) or (A14-2) or (A14-3) (described in, for example, WO-A-2019/074110). Besides these linkers, examples of the non-nucleotide linker include the linkers described in WO-A-2012/005368, WO-A-2018/182008, or WO-A-2019/074110. 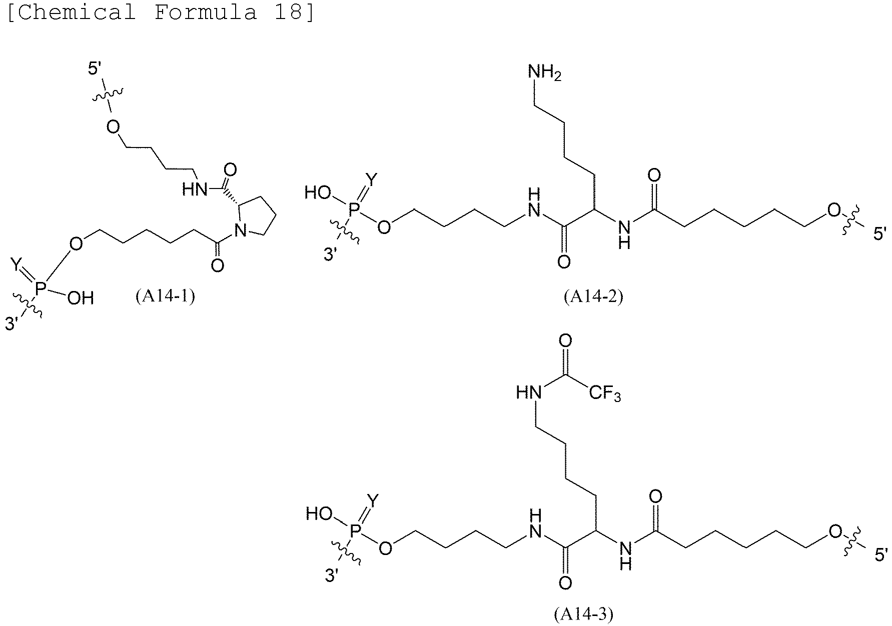 (in the formula, Y is as described above).

Nucleotides and amidites where the R group in the formula (13) and the R' group in the formula (5) are substituents other than a hydroxyl group can also be produced from nucleosides synthesized by known methods as described in JP-B-3745226 and the like, and WO-A-2001/053528 or JP-A-2014-221817, and the known methods cited therein, and furthermore, with the use of those available as commercial products, can be produced in accordance with the methods described in examples as described later or by the method to which appropriate changes are made.

G⁴ represents a hydrogen atom, an alkali metal ion, an ammonium ion, an alkylammonium ion, or a hydroxyalkylammonium ion. Examples of the alkali metal ion include a sodium ion and a lithium ion. In addition, as the alkylammonium ion, specific examples of the alkyl group include a methyl group, an ethyl group, a n-propyl group, an isopropyl group, a n-butyl group, an isobutyl group, a tert-butyl group, a n-pentyl group, an isopentyl group, and a hexyl group, and more specific examples thereof include a diethylammonium ion, a triethylammonium ion, a tetrabutylammonium ion, a hexylammonium ion, and a dibutylammonium ion. In addition, as the hydroxyalkylammonium ion, specific examples of the hydrokyalkyl moiety include hydroxymethyl, hydroxyethyl, hydroxy-n-propyl, hydroxyisopropyl, hydroxy-n-butyl, and trishydroxymethyl, and more specific examples of the hydroxyalkylammonium ion include a trishydroxymethylammonium ion. G⁴ preferably represents a hydrogen atom.

G⁵ represents a hydrogen atom or a protecting group for the hydroxyl group, and when G⁵ represents a protecting group, G¹ also represents the same protecting group. G⁵ is a hydrogen atom in the case of being deprotected, but the nucleotide compound in that case is also subjected to the series of steps for the nucleic acid elongation reaction.

Y is preferably an oxygen atom.

As for W₁ and X₁, preferably, W₁ represents an OZ group, and X₁ represents an R group.

As for W₂ and X₂, preferably, W₂ represents a hydroxyl group, and X₂ represents an R group.

W₃ and X₃ preferably each independently represent a hydroxyl group.

R' is preferably a hydroxyl group.

For the synthesis of the nucleic acid compound by the amidite method from the steps (1) to (5), a nucleic acid elongation reaction can be developed in accordance with a generally known method (for example, the method described in JP-B-5157168 or JP-B-5554881), except for the deprotecting step according to the present invention in the step (1) or step (5) in the scheme of Fig. 1. Each step will be described below.

### (Nucleic Acid Elongation Reaction)

In this specification, the "nucleic acid elongation reaction" means a reaction of sequentially binding nucleotides via phosphodiester bonds to elongate an oligonucleotide. The nucleic acid elongation reaction can be developed in accordance with the procedure of a common phosphoramidite method. The nucleic acid elongation reaction may be developed with the use of an automatic nucleic acid synthesizer in which a phosphoramidite method is employed, or the like.

The chain length of the nucleic acid oligomer may be, for example, 20 mer or more (that is, n ≥ 19), 40 mer or more (that is, n ≥ 39), 50 mer or more (that is, n ≥ 49), 60 mer or more (hat is, n ≥ 59), 80 mer or more (that is, n ≥ 79), 100 mer or more (that is, n ≥ 99), 2 to 200 mer (that is, 1 ≤ n ≤ 199), 10 to 150 mer (that is, 9 ≤ n ≤ 149), or 15 to 110 mer (that is, 14 ≤ n ≤ 109).

The deprotecting step of the step (1) is a step of deprotecting the protecting group of the 5'-hydroxyl group at the terminal of the oligonucleotide chain supported on the solid support. As a common protecting group, a 4,4'-dimethoxytrityl group (DMTr group), a 4-monomethoxytrityl group, or a 4,4',4"-trimethoxytrityl group is used. The deprotection can be performed with the use of an acid. Examples of the acid for the deprotection include trifluoroacetic acid, trichloroacetic acid, trifluoromethanesulfonic acid, dichloroacetic acid, methanesulfonic acid, hydrochloric acid, acetic acid, and p-toluenesulfonic acid.

The coupling step of the step (2) is a reaction in which a nucleoside phosphoramidite represented by the following formula (13) listed in the Scheme A of Fig. 1 is bound to the 5'-hydroxyl group at the terminal of the oligonucleotide chain deprotected by the deprotecting step. It is to be noted that an amidite compound represented by the formula (13) or (A9) to (A12) is used as the phosphoramidite used for the nucleic acid elongation. In addition, other examples of the phosphoramidite that can be used include 2'-OMe, 2'-F, a 2'-O-tert-butyldimethylsilyl group, a 2'-O-methoxyethyl group, 2'-H, and a 2'-fluoro-2'-deoxy-β-D-arabinofuranosyl group. As the nucleoside phosphoramidite, a 5'-hydroxyl group protected with a protecting group (e.g., DMTr group) is used. The coupling step can be performed with the use of an activating agent or a coupling agent that activates the nucleoside phosphoramidite. Examples of the activating agent or coupling agent include a 5-benzylthio-1H-tetrazole (BTT) (also referred to as a 5-benzylmercapto-1H-tetrazole), a 1H-tetrazole, a 4,5-dicyanoimidazole (DCI), a 5-ethylthio-1H-tetrazole (ETT), an N-methylbenzimidazolium triflate (N-MeBIT), a benzimidazolium triflate (BIT), an N-phenylimidazolium triflate (N-PhIMT), an imidazolium triflate (IMT), a 5-nitrobenzimidazolium triflate (NBT), a 1-hydroxybenzotriazole (HOBT), or a 5-(bis -3,5-trifluoromethylphenyl)-1H-tetrazole.

The nucleoside phosphoramidite represented by the formula (13) (hereinafter, referred to as an amidite) shown in the Scheme A of Fig. 1 is as follows:
A compound represented by the following formula: 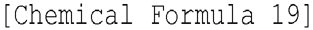 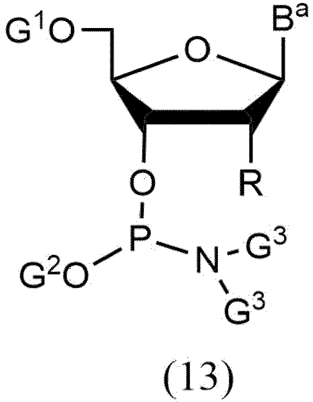 (in the formula,
G¹, G², G³, B^{a}, and R are as described above) .

After the coupling step, the unreacted 5'-hydroxyl group may be subjected to capping appropriately. The capping can be performed with the use of a known capping solution such as an acetic anhydride-tetrahydrofuran solution or a phenoxyacetic anhydride/N-methylimidazole solution.

The oxidizing step of the step (3) is a step of converting the phosphite group formed in accordance with the coupling step into a phosphate group or a thiophosphate group. This step is a reaction of converting trivalent phosphorus into pentavalent phosphorus with the use of an oxidizing agent, and can be performed by reacting the oxidizing agent with the oligonucleic acid derivative supported on the solid support.

In the case of converting the phosphite group into a phosphate group, for example, iodine can be used as the "oxidizing agent". The oxidizing agent can be prepared so as to have a concentration of 0.005 to 2 M, and used. Water can be used as an oxygen source for the oxidation, and a pyridine, an N-methylimidazole (NMI), an N-methylmorpholine, a triethylamine, or the like can be used as a base for allowing the reaction to proceed. In addition, the solvent is not particularly limited as long as the solvent is not involved in the reaction, and examples thereof include acetonitrile, tetrahydrofuran (THF), or mixed solvents in arbitrary proportions thereof. For example, iodine/water/pyridine/acetonitrile, or iodine/water/pyridine, or iodine/water/pyridine/NMI, or iodine/water/pyridine/THF can be used. The reaction temperature is preferably 5°C to 50°C. Typically, the reaction time is appropriately 1 minute to 30 minutes. The amount of the reagent used is preferably 1 to 100 mol, and more preferably 1 to 10 mol with respect to 1 mol of the compound supported on the solid support.

In the case of converting the phosphite triester group to a thiophosphate triester group, for example, sulfur, a 3H-1,2-benzodithiol-3-one-1,1-dioxide (Beaucage reagent), a 3-amino-1,2,4-dithiazole-5-thione (ADTT), a 5-phenyl-3H-1,2,4-dithiazole-3-one (POS), an [(N,N-dimethylaminomethylidene)amino]-3H-1,2,4-dithiazoline-3-thione (DDTT), and a phenyl acetyl disulfide (PADS) can be used as the "oxidizing agent". The oxidizing agent can be diluted with an appropriate solvent so as to have a concentration of 0.001 to 2 M, and used. The solvent for use in the reaction is not particularly limited as long as the solvent is not involved in the reaction, and examples thereof include dichloromethane, acetonitrile, pyridine, or mixed solvents in arbitrary proportions thereof. The oxidizing step may be performed after the capping operation, or conversely, the capping operation may be performed after the oxidizing step, and this order is not limited.

In the step (5-1), the protecting group for the hydroxyl group at the 5'-position of the nucleotide introduced at the end of the elongation may be used for column purification with the protecting group for the hydroxyl group at the 5'-position as a tag after cutting out from the solid support and deprotecting the protecting group as described later, or the protecting group for the hydroxyl group at the 5'-position may be deprotected after column purification.

In the step (5-2), in the step of deprotecting the phosphate protecting group, after completing the synthesis of the nucleic acid that has a desired sequence, an amine compound is allowed to act for deprotecting the protecting group of the phosphate moiety. Examples of the amine compound include a diethylamine described in JP-B-4705716.

The cutting out of the nucleic acid oligomer elongated to a desired chain length on the solid support from the solid support in the step (5-2) is typically performed with the use of concentrated ammonia water as a cutting agent.

Further, for example, the oligonucleotide chain is broken from the solid support with the use of ammonia, an amine compound, or the like, and then collected. Examples of the amine compound include a methylamine, an ethylamine, an isopropylamine, an ethylenediamine, and a diethylamine.

In the step (5-3), the protecting group for the hydroxyl group at the 2'-position or 3'-position of the ribose of the nucleic acid compound (4) cut out from the solid support in the step (5-2) can be removed in accordance with the method described in WO-A-2006/022323), WO-A-2013/027843, or WO-A-2019/208571, and a deprotected nucleic acid oligomer (5) can be obtained.

Examples of the nucleic acid oligomer that can be produced with the use of the production method according to the present invention include, but not be considered limited to, nucleic acid oligomers with a nucleoside included therein being RNA, DNA, RNA having a 2'-O-MOE, a 2'-O-Me, or a 2'-F, and LNA. Examples of various nucleosides are provided, for example, as described in Xiulong, Shen et al., Nucleic Acids Research, 2018, Vol. 46, No. 46, 1584-1600, and Daniel O'Reilly et al., Nucleic Acids Research, 2019, Vol. 47, No. 2, 546-558. Preferably, the nucleic acid oligomer produced by the method according to the present invention is RNA.

Typical examples of the nucleic acid oligomer that can be used in the production method according to the present invention are, in addition to the examples described in examples, shown in the following examples, but are not to be considered limited thereto.

Hereinafter, in the descriptions of sequences, U represents a uridine, C represents a cytidine, A represents an adenosine, and G represents a guanosine.

Examples thereof include the nucleic acid oligomers that have the following sequences (A) and (B), described in WO-A-2019/060442.
Sequence (A): 5'-AUGGAAUmACUCUUGGUUmACdTdT-3' (Antisense) (SEQ ID NO: 1) 21 mer
Sequence (B): 5'-GUmAACmCmAAGAGUmAUmUmCmCmAUmdTdT-3' (Sense) (SEQ ID NO: 2) 21 mer

In the sequences (A) and (B), Um represents a 2'-O-methyluridine, Cm represents a 2'-O-methylcytidine, and dT represents a thymidine.

The examples include the nucleic acid oligomers (see page 553) described in Daniel O'Reilly et al., Nucleic Acids Research, 2019, Vol. 47, No. 2, 546-558. Typical examples thereof include a nucleic acid oligomer that has the following sequence (C).
Sequence (C): 5'-AGAGCCAGCCUUCUUAUUGUUUUAGAGCUAUGCUGU-3' (SEQ ID NO: 3) 36 mer

The examples include the nucleic acid oligomers described in JP-B-4965745. Typical examples thereof include a nucleic acid oligomers that has the following sequence (D).
Sequence (D): 5'-CCAUGAGAAGUAUGACAACAGCC-P-GGCUGUUGUCAUACUUCUCAUGGUU-3' (SEQ ID NOs: 4 and 5) 49 mer

In the sequence (D), "P" is represented by a partial structure delimited by a wavy line in the following formula (A5) .

It is to be noted that the description of SEQ ID NO: 4 in the sequence listing indicates the base sequence of the following sequence (D1) from the 5' terminal of the sequence (D) to before "P" thereof, and the description of SEQ ID NO: 5 therein indicates the base sequence of the following sequence (D2) from after "P" of the sequence (D) to the 3' terminal thereof.
Sequence (D1): 5'-CCAUGAGAAGUAUGACAACAGCC-3' (SEQ ID NO: 4) 23 mer
Sequence (D2): 5'-GGCUGUUGUCAUACUUCUCAUGGUU-3' (SEQ ID NO: 5) 25 mer

The examples include the nucleic acid oligomer that has the following sequence (E), described in Nucleic Acids Research, 2019, Vol. 47, No. 2: 547.

Sequence (E): 5'-

ACAGCAUAGCAAGUUAAAAUAAGGCUAGUCCGUUAUCAACUUGAAAAAGUGGCACCGAG

UCGGUGCU-3' (SEQ ID NO: 6) 67 mer

The examples include the nucleic acid oligomer that has the following sequence (F), described in JP-W-2015-523856, page 173.

Sequence (F): 5'-
GUUUUCCCUUUUCAAAGAAAUCUCCUGGGCACCUAUCUUCUUAGGUGCCCUCCCUUGUU
UAAACCUGACCAGUUAACCGGCUGGUUAGGUUUUU-3' (SEQ ID NO: 7) 94
mer

The examples include the nucleic acid oligomers described in JP-W-2017-537626. Typical examples thereof include nucleic acid oligomers that have the following sequences (G), (H), (I), and (J).

Sequence (G): 5'-
AGUCCUCAUCUCCCUCAAGCGUUUUAGAGCUAGUAAUAGCAAGUUAAAAUAAGGCUAGU
CCGUUAUCAACUUGAAAAAGUGGCACCGAGUCGGUGCUUUU-3' (SEQ ID NO: 8)
100 mer

Sequence (H): 5'-

GCAGAUGUAGUGUUUCCACAGUUUAAGAGCUAUGCUGGAAACAGCAUAGCAAGUUUAAA

UAAGGCUAGUCCGUUAUCAACUUGAAAAAGUGGCACCGAGUCGGUGCUUUUUUU-3'

(SEQ ID NO: 9) 113 mer

Sequence (I): 5'-

dAdGdTdCdCdTdCdAdTdCdTdCdCdCdTdCdAdAdGdCGUUUAAGAGCUAUGCUGGU

AACAGCAUAGCAAGUUUAAAUAAGGCUAGUCCGUUAUCAACUUGAAAAAGUGGCACCGA

GUCGGUGCUUUUUUU-3' (SEQ ID NO: 10) 113 mer

In the sequence (I), dT represents a thymidine, dC represents a 2'-deoxycytidine, dA represents a 2'-deoxyadenosine, and dG represents a 2'-deoxyguanosine.

Sequence (J): 5'-

AmsGmsUmsCCUCAUCUCCCUCAAGCGUUUAAGAGCUAUGCUGGUAACAGCAUAGC

AAGUUUAAAUAAGGCUAGUCCGUUAUCAACUUGAAAAAGUGGCACCGAGUCGGUGCUUU

UmsUmsUmsU-3' (SEQ ID NO: 11) 113 mer

In the sequence (J), Um represents a 2'-O-methyluridine, Am represents a 2'-O-methyladenosine, Gm represents a 2'-O-methylguanosine, and s represents a phosphorothioate modification.

### EXAMPLES

Hereinafter, the present invention will be described in more detail with reference to examples, but the present invention is not to be considered limited to these examples.

### <Measurement Method>

First, here are various measurement methods used in the following tests.

The oligonucleotide purity was measured with the use of HPLC.

The HPLC measurement conditions are shown in Table 1 below.

### (Measurement Method 1: Measurement of Oligonucleotide Purity)

**[Table 1]**

| | |
|---|---|
| Column | DNAPac^{™}, PA200, 4.0 × 250 mm |
| Flow Rate | 1.0 mL/min |
| Detection Wavelength | 260 nm |
| Mobile Phase A | 25 mM Tris-HCl buffer (pH = 8.0), 10% CH₃CN, 6M Urea Water |
| Mobile Phase B | 500 mM NaClO₄, 25 mM Tris-HCl buffer (pH = 8.0), 10% CH₃CN, 6M Urea Water |
| Gradient Condition | B conc. 20% (0 min) - 60% (60 min) - 90% (60.01 min) - 90% (65 min) - 20% (65.01 min) - 20% (80 min) |
| Column Temperature | 80°C |

### (Measurement Method 2: Measurement of Oligonucleotide Yield)

The OD₂₆₀ of the crude product was measured. The OD₂₆₀ represents the absorbance at UV 260 nm per 10 mm optical path length in a 1 mL solution (pH = 7.5). Typically, RNA is known to be 1 OD = 40 µg, and thus, based on the measurement value of OD₂₆₀, the yield was calculated.

### (Measurement Method 3: Measurement of Formaldehyde Concentration)

Examples of the method of measuring the formaldehyde concentration in the trichloroacetic acid solution include a high-performance liquid chromatographic method. In the high-performance liquid chromatographic method, formaldehyde and acetylacetone are reacted, the amount of 3,5-diacetyl-1,4-dihydrolutidine obtained is measured, and the concentration of the formaldehyde is calculated.

### <Preparation of Trichloroacetic Acid Solution>

Trichloroacetic acid solutions that were different in formaldehyde concentration, used in the following tests, were prepared by preparing a trichloroacetic acid solution that was low formaldehyde concentration in advance and adding an aqueous formaldehyde solution to the obtained trichloroacetic acid solution.

### <Solid-phase Synthesis of Oligonucleotide>

Sequence (I): 5'-GGCACCGAGUCGGUGCUUUU-3' (SEQ ID NO: 12) 20 mer

Sequence (II): 5'-
AAGGCUAGUCCGUUAUCAACUUGAAAAAGUGGCACCGAGUCGGUGCUUUU-3'(SEQ
ID NO: 13) 50 mer

Sequence (III): 5'-
AUAACUCAAUUUGUAAAAAAGUUUUAGAGCUAGAAAUAGCAAGUUAAAAUAAGGCUAGU
CCGUUAUCAACUUGAAAAAGUGGCACCGAGUCGGUGCUUUU-3' (SEQ ID NO:
14) 100 mer

In the sequences (I), (II), and (III), "A" is represented by a partial structure delimited by a wavy line in the following formula (A1). "C" is represented by a partial structure delimited by a wavy line in the following formula (A2). "G" is represented by a partial structure delimited by a wavy line in the following formula (A3). U is represented by a partial structure delimited by a wavy line in the following formula (A4). Further, the "U" at the 3'-terminal is represented by a partial structure delimited by a wavy line in the following formula (A8). In addition, in the sequence (I), the "G" at the 5'-terminal is represented by a partial structure delimited by a wavy line in the following formula (A6), and in the sequences (II) and (III), the "A" at the 5'-terminal is represented by a partial structure delimited by a wavy line in the following formula (A7).

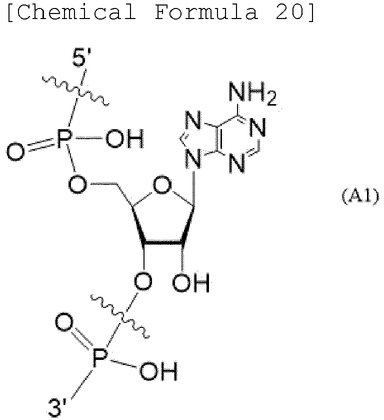

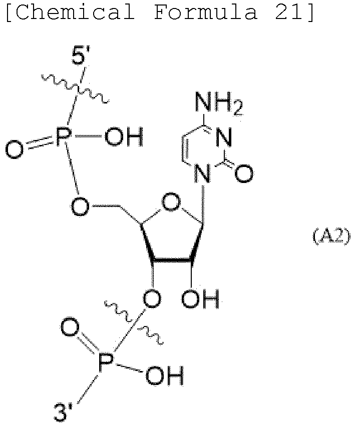

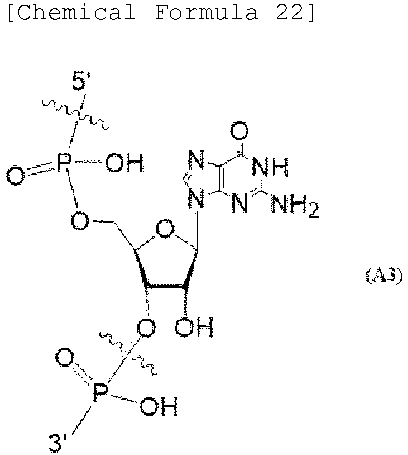

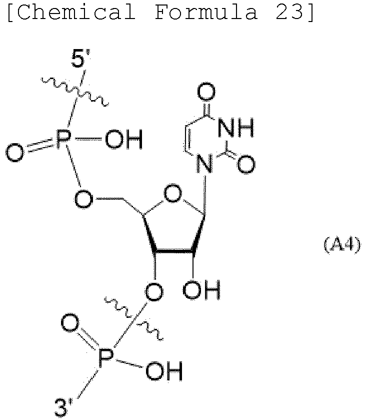

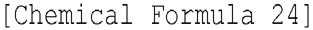

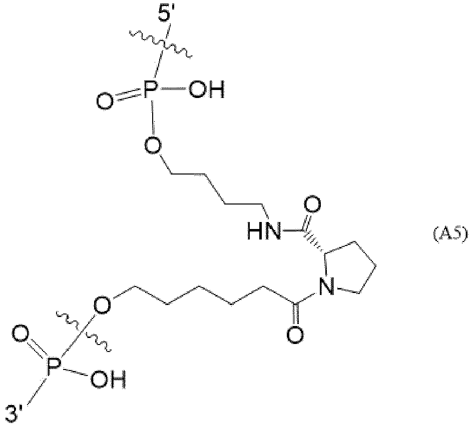

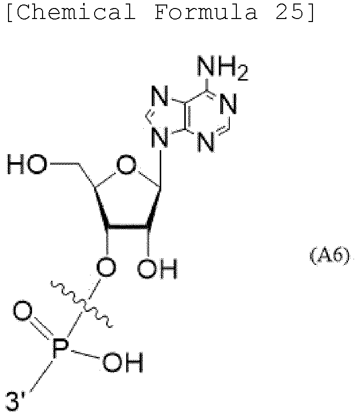

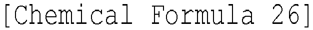

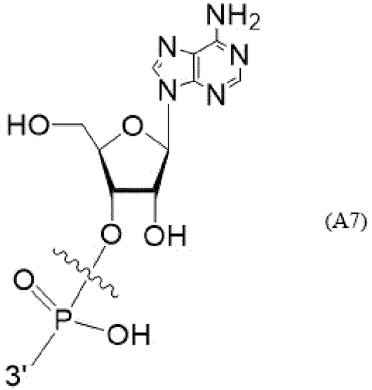

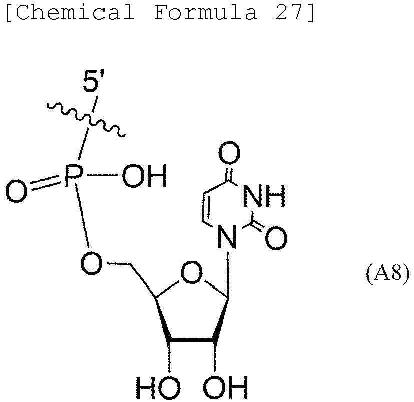

With the use of Controlled Pore Glass (CPG) as a solid support and NTS M-4MX-E (manufactured by NIHON TECHNO SERVICE CO., LTD.) as a nucleic acid synthesizer, an oligonucleotide composed of the sequences (I), (II) and (III) was synthesized from the 3' side toward the 5' side by a phosphoramidite solid-phase synthesis method. The synthesis was carried out on an about 1 µmol scale. In addition, for the synthesis, the uridine EMM amidite (A11) described in Example 2 of US2012/0035246, the cytidine EMM amidite (A9) described in Example 3 thereof, the adenosine EMM amidite (A12) described in Example 4 thereof, and the guanosine EMM amidite (A10) described in Example 5 thereof were used, a 3% trichloroacetic acid toluene solution was used as a deblocking solution, a 5-benzylmercapto-1H-tetrazole was used as a coupling agent, an iodine solution was used as an oxidizing agent, and a phenoxyacetic anhydride solution and an N-methylimidazole solution were used as capping solutions.

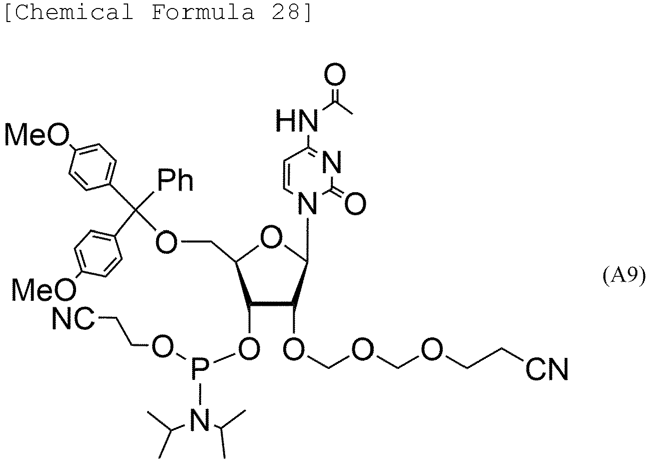

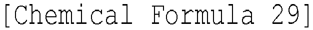

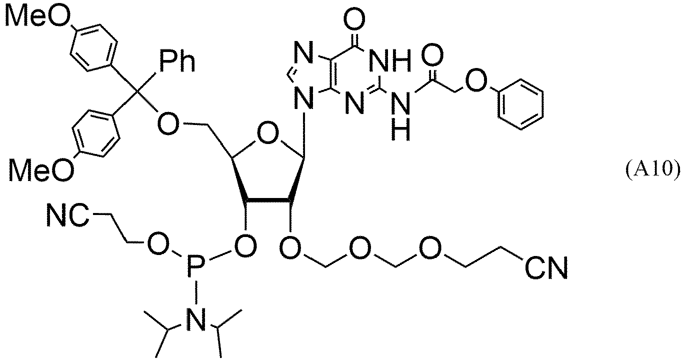

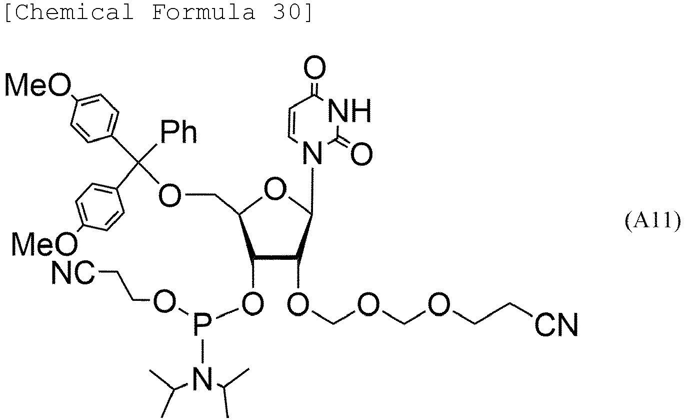

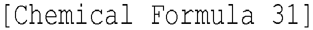

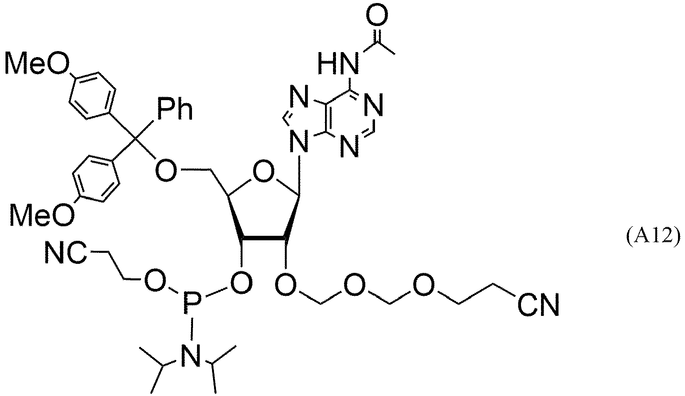

Next, specific examples of producing the oligonucleotides (nucleic acid oligomers) produced by the production method according to the present invention will be described. In this regard, the oligonucleotides produced by the production method according to the present invention in the following examples are oligonucleotides that have the sequences (I), (II), or (III).

In addition, the uridine derivative described in the following examples and comparative examples means a compound represented by the following structural formula. The circle illustrated in the following structural formula is intended to schematically show a CPG.

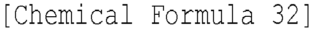

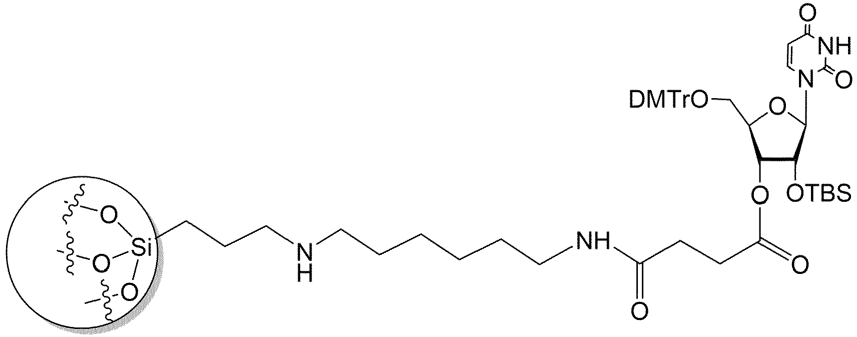

### (Example 1)

With the use of Controlled Pore Glass (CPG) with 0.99 µmol of the uridine derivative supported thereon and the amidite represented by the formula (A9), formula (A10), formula (A11), or formula (A12), the nucleic acid oligomer represented by the sequence (I) was automatically synthesized from the 3' side toward the 5' side by NTS M 4MX-E (manufactured by NIHON TECHNO SERVICE CO., LTD.). In the automatic synthesis procedure, first, a 3% trichloroacetic acid toluene solution was sent to the CPG to deprotect the trityl protecting group at the 5'-position. In this regard, the formaldehyde concentration in the trichloroacetic acid solution used was allowed to be measured by the Measurement Method 3, and the molar ratio of the formaldehyde to the trichloroacetic acid in the trichloroacetic acid solution (formaldehyde mol/trichloroacetic acid mol) was 33 × 10⁻⁷. Subsequently, various amidites and a 5-benzylmercapto-1H-tetrazole as a coupling agent were sent to the CPG to allow a coupling reaction to proceed for the hydroxyl group at the 5'-position. Subsequently, an oxidizing solution containing 50 mM iodine was sent to convert the phosphite group into a phosphate group. Subsequently, with the use of a 0.1 M phenoxyacetic anhydride acetonitrile solution and a 10% N-methylimidazole/10% 2,6-lutidine acetonitrile solution as capping solutions, reactive sites where no coupling proceeded were subjected to capping. Further, after repeating these steps 19 times in total, the protecting group (DMTr group) of the 5'-terminal base was deprotected with a 3% trichloroacetic acid toluene solution to synthesize a nucleic acid oligonucleotide with a sequence represented by the sequence (I) on the CPG carrier. Thereafter, 1.5 mL of 28% ammonia water and 0.5 mL of ethanol were allowed to flow into the CPG carrier with 0.99 µmol of the oligonucleotide supported thereon, and the mixture was kept at a temperature of 40°C for 4 hours to release the nucleic acid oligomer from the solid support, and then concentrated to remove the solvent. Then, in 1.5 mL of a dimethyl sulfoxide, the free oligonucleotide was dissolved, 1.0 mL of acetonitrile, 20 µL of a nitromethane, and a stirrer were then put, and 2.08 mL of a 1 M solution of tetra-n-butylammonium fluoride (TBAF) in a dimethyl sulfoxide, subjected to a dehydration treatment with a molecular sieve 4A, was allowed to flow at room temperature under stirring with the stirrer, and the mixture was kept at a temperature of 33°C for 4 hours to deprotect the 2'-EMM protecting group. Thereafter, a product of the nucleic acid oligomer was obtained by the operation of precipitation. As a result of measuring the purity of the oligonucleotide for the obtained product with the use of the method described in the Measurement Method 1, the purity was 72.6%. In addition, when the yield of oligonucleotides was measured with the use of the method described in the Measurement Method 2, the yield was 4369 µg, which was 4413 µg in terms of yield per CPG with 1.00 µmol of the uridine derivative supported thereon. The results are shown in Table 2.

### (Example 2)

A nucleic acid oligomer in accordance with the sequence (I) was obtained in the same manner as in the experiment according to Example 1, except for the use of Controlled Pore Glass (CPG) with 1.00 µmol of the uridine derivative supported thereon and a 3% trichloroacetic acid toluene solution in which the molar ratio of the formaldehyde to the trichloroacetic acid (formaldehyde mol/trichloroacetic acid mol) was 11 × 10⁻⁵. As a result of measuring the purity of the oligonucleotide with the use of the method described in the Measurement Method 1, the purity of the product was 71.4%. In addition, when the yield of oligonucleotides was measured with the use of the method described in the Measurement Method 2, the yield was 4236 µg in terms of yield per CPG with 1.00 µmol of the uridine derivative supported thereon. The results are shown in Table 2.

### (Reference Example 1)

A nucleic acid oligomer in accordance with the sequence (I) was obtained in the same manner as in the experiment according to Example 1, except for the use of Controlled Pore Glass (CPG) with 0.97 µmol of the uridine derivative supported thereon and a 3% trichloroacetic acid toluene solution in which the molar ratio of the formaldehyde to the trichloroacetic acid (formaldehyde mol/trichloroacetic acid mol) was 26 × 10⁻⁵. As a result of measuring the purity of the oligonucleotide with the use of the method described in the Measurement Method 1, the purity of the product was 69.7%. In addition, when the yield of oligonucleotides was measured with the use of the method described in the Measurement Method 2, the yield was 3982 µg, which was 4105 µg in terms of yield per CPG with 1.00 µmol of the uridine derivative supported thereon. The results are shown in Table 2.

### (Example 3)

With the use of Controlled Pore Glass (CPG) with 1.01 µmol of the uridine derivative supported thereon and the amidite represented by the formula (A9), formula (A10), formula (A11), or formula (A12), the nucleic acid oligomer represented by the sequence (II) was automatically synthesized from the 3' side toward the 5' side by NTS M 4MX-E (manufactured by NIHON TECHNO SERVICE CO., LTD.). In the automatic synthesis procedure, first, a 3% trichloroacetic acid toluene solution was sent to the CPG to deprotect the trityl protecting group at the 5'-position. In this regard, the formaldehyde concentration in the trichloroacetic acid solution used was allowed to be measured by the Measurement Method 3, and the molar ratio of the formaldehyde to the trichloroacetic acid in the trichloroacetic acid solution (formaldehyde mol/trichloroacetic acid mol) was 33 × 10⁻⁷. Subsequently, various amidites and a 5-benzylmercapto-1H-tetrazole as a coupling agent were sent to the CPG to allow a coupling reaction to proceed for the hydroxyl group at the 5'-position. Subsequently, an oxidizing solution containing 50 mM iodine was sent to convert the phosphite group into a phosphate group. Subsequently, with the use of a 0.1 M phenoxyacetic anhydride acetonitrile solution and a 10% N-methylimidazole/10% 2,6-lutidine acetonitrile solution as capping solutions, reactive sites where no coupling proceeded were subjected to capping. Further, after repeating these steps 49 times in total, the protecting group (DMTr group) of the 5'-terminal base was deprotected with a 3% trichloroacetic acid toluene solution to synthesize a nucleic acid oligonucleotide with a sequence represented by the sequence (II) on the CPG carrier. Thereafter, 1.5 mL of 28% ammonia water and 0.5 mL of ethanol were allowed to flow into the CPG carrier with 1.01 µmol of the oligonucleotide supported thereon, and the mixture was kept at a temperature of 40°C for 4 hours to release the nucleic acid oligomer from the solid support, and then concentrated to remove the solvent. Then, in 1.5 mL of a dimethyl sulfoxide, the free oligonucleotide was dissolved, 1.0 mL of acetonitrile, 20 µL of a nitromethane, and a stirrer were then put, and 2.08 mL of a 1 M solution of tetra-n-butylammonium fluoride (TBAF) in a dimethyl sulfoxide, subjected to a dehydration treatment with a molecular sieve 4A, was allowed to flow at room temperature under stirring with the stirrer, and the mixture was kept at a temperature of 33°C for 4 hours to deprotect the 2'-EMM protecting group. Thereafter, a product of the nucleic acid oligomer was obtained by the operation of precipitation. As a result of measuring the purity of the oligonucleotide for the obtained product with the use of the method described in the Measurement Method 1, the purity was 43.1%. In addition, when the yield of oligonucleotides was measured with the use of the method described in the Measurement Method 2, the yield was 9941 µg, which was 9843 µg in terms of yield per CPG with 1.00 µmol of the uridine derivative supported thereon. The results are shown in Table 2.

### (Example 4)

A nucleic acid oligomer in accordance with the sequence (II) was obtained in the same manner as in the experiment according to Example 3, except for the use of Controlled Pore Glass (CPG) with 1.01 µmol of the uridine derivative supported thereon and a 3% trichloroacetic acid toluene solution in which the molar ratio of the formaldehyde to the trichloroacetic acid (formaldehyde mol/trichloroacetic acid mol) was 11 × 10⁻⁵. As a result of measuring the purity of the oligonucleotide with the use of the method described in the Measurement Method 1, the purity of the product was 36.5%. In addition, when the yield of oligonucleotides was measured with the use of the method described in the Measurement Method 2, the yield was 9710 µg, which was 9614 µg in terms of yield per CPG with 1.00 µmol of the uridine derivative supported thereon. The results are shown in Table 2.

### (Reference Example 2)

A nucleic acid oligomer in accordance with the sequence (II) was obtained in the same manner as in the experiment according to Example 3, except for the use of Controlled Pore Glass (CPG) with 1.02 µmol of the uridine derivative supported thereon and a 3% trichloroacetic acid toluene solution in which the molar ratio of the formaldehyde to the trichloroacetic acid (formaldehyde mol/trichloroacetic acid mol) was 26 × 10⁻⁵. As a result of measuring the purity of the oligonucleotide with the use of the method described in the Measurement Method 1, the purity of the product was 33.2%. In addition, when the yield of oligonucleotides was measured with the use of the method described in the Measurement Method 2, the yield was 9158 µg, which was 8978 µg in terms of yield per CPG with 1.00 µmol of the uridine derivative supported thereon. The results are shown in Table 2.

### (Example 5)

With the use of Controlled Pore Glass (CPG) with 1.00 µmol of the uridine derivative supported thereon and the amidite represented by the formula (A9), formula (A10), formula (A11), or formula (A12), the nucleic acid oligomer represented by the sequence (III) was automatically synthesized from the 3' side toward the 5' side by NTS M 4MX-E (manufactured by NIHON TECHNO SERVICE CO., LTD.). In the automatic synthesis procedure, first, a 3% trichloroacetic acid toluene solution was sent to the CPG to deprotect the trityl protecting group at the 5'-position. In this regard, the formaldehyde concentration in the trichloroacetic acid solution used was allowed to be measured by the Measurement Method 3, and the molar ratio of the formaldehyde to the trichloroacetic acid in the trichloroacetic acid solution (formaldehyde mol/trichloroacetic acid mol) was 33 × 10⁻⁷. Subsequently, various amidites and a 5-benzylmercapto-1H-tetrazole as a coupling agent were sent to the CPG to allow a coupling reaction to proceed for the hydroxyl group at the 5'-position. Subsequently, an oxidizing solution containing 50 mM iodine was sent to convert the phosphite group into a phosphate group. Subsequently, with the use of a 0.1 M phenoxyacetic anhydride acetonitrile solution and a 10% N-methylimidazole/10% 2,6-lutidine acetonitrile solution as capping solutions, reactive sites where no coupling proceeded were subjected to capping. Further, after repeating these steps 99 times in total, the protecting group (DMTr group) of the 5'-terminal base was deprotected with a 3% trichloroacetic acid toluene solution to synthesize a nucleic acid oligonucleotide with a sequence represented by the sequence (III) on the CPG carrier. Thereafter, 1.5 mL of 28% ammonia water and 0.5 mL of ethanol were allowed to flow into the CPG carrier with 1.00 µmol of the oligonucleotide supported thereon, and the mixture was kept at a temperature of 40°C for 4 hours to release the nucleic acid oligomer from the solid support, and then concentrated to remove the solvent. Then, in 1.5 mL of a dimethyl sulfoxide, the free oligonucleotide was dissolved, 1.0 mL of acetonitrile, 20 µL of a nitromethane, and a stirrer were then put, and 2.08 mL of a 1 M solution of tetra-n-butylammonium fluoride (TBAF) in a dimethyl sulfoxide, subjected to a dehydration treatment with a molecular sieve 4A, was allowed to flow at room temperature under stirring with the stirrer, and the mixture was kept at a temperature of 33°C for 4 hours to deprotect the 2'-EMM protecting group. Thereafter, a product of the nucleic acid oligomer was obtained by the operation of precipitation. As a result of measuring the purity of the oligonucleotide for the obtained product with the use of the method described in the Measurement Method 1, the purity was 32.8%. In addition, when the yield of oligonucleotides was measured with the use of the method described in the Measurement Method 2, the yield was 15722 µg in terms of yield per CPG with 1.00 µmol of the uridine derivative supported thereon. The results are shown in Table 2.

### (Example 6)

A nucleic acid oligomer in accordance with the sequence (III) was obtained in the same manner as in the experiment according to Example 5, except for the use of Controlled Pore Glass (CPG) with 1.01 µmol of the uridine derivative supported thereon and a 3% trichloroacetic acid toluene solution in which the molar ratio of the formaldehyde to the trichloroacetic acid (formaldehyde mol/trichloroacetic acid mol) was 11 × 10⁻⁵. As a result of measuring the purity of the oligonucleotide with the use of the method described in the Measurement Method 1, the purity of the product was 30.9%. In addition, when the yield of oligonucleotides was measured with the use of the method described in the Measurement Method 2, the yield was 14754 µg, which was 14608 µg in terms of yield per CPG with 1.00 µmol of the uridine derivative supported thereon. The results are shown in Table 2.

### (Reference Example 3)

A nucleic acid oligomer in accordance with the sequence (III) was obtained in the same manner as in the experiment according to Example 5, except for the use of Controlled Pore Glass (CPG) with 0.97 µmol of the uridine derivative supported thereon and a 3% trichloroacetic acid toluene solution in which the molar ratio of the formaldehyde to the trichloroacetic acid (formaldehyde mol/trichloroacetic acid mol) was 26 × 10⁻⁵. As a result of measuring the purity of the oligonucleotide with the use of the method described in the Measurement Method 1, the purity of the product was 26.7%. In addition, when the yield of oligonucleotides was measured with the use of the method described in the Measurement Method 2, the yield was 13283 µg, which was 13694 µg in terms of yield per CPG with 1.00 µmol of the uridine derivative supported thereon. The results are shown in Table 2.

### (Example 7)

To 30 g of a trichloroacetic acid in which the molar ratio of the formaldehyde to the trichloroacetic acid (formaldehyde mol/trichloroacetic acid mol) was 25 × 10⁻⁵, 300 mL of a toluene was added, and the toluene and the formaldehyde were azeotropically distilled away at 40°C with the use of an evaporator to obtain 34 g of a colorless oily trichloroacetic acid solution. When the formaldehyde included in the obtained trichloroacetic acid solution was analyzed by the method described in the Measurement Method 3, the molar ratio of the formaldehyde to the trichloroacetic acid was 25 × 10⁻⁷.

**[Table 2]**

| | Synthesis sequence | Molar Ratio of formaldehyde to trichloroacetic acid | Yield (µg/µmol) | Product purity (%) | Pure yield (yield × product purity) | Relative quantity of pure yield |
|---|---|---|---|---|---|---|
| Example 1 | Sequence (I) | 33 × 10⁻⁷ | 4413 | 72.6 | 3204 | 1.12 |
| Example 2 | Sequence (I) | 11 × 10⁻⁵ | 4236 | 71.4 | 3025 | 1.06 |
| Reference Example 1 | Sequence (I) | 26 × 10⁻⁵ | 4105 | 69.7 | 2861 | 1.00 |
| Example 3 | Sequence (II) | 33 × 10⁻⁷ | 9843 | 43.1 | 4242 | 1.42 |
| Example 4 | Sequence (II) | 11 × 10⁻⁵ | 9614 | 36.5 | 3509 | 1.18 |
| Reference Example 2 | Sequence (II) | 26 × 10⁻⁵ | 8978 | 33.2 | 2981 | 1.00 |
| Example 5 | Sequence (III) | 33 × 10⁻⁷ | 15722 | 32.8 | 5157 | 1.41 |
| Example 6 | Sequence (III) | 11 × 10⁻⁵ | 14608 | 30.9 | 4514 | 1.23 |
| Reference Example 3 | Sequence (III) | 26 × 10⁻⁵ | 13694 | 26.7 | 3656 | 1.00 |

From the foregoing results in Table 2, in the case of using the trichloroacetic acid solution according to the present invention in which the formaldehyde concentration was equal to or less than a certain level, a nucleic acid oligomer was obtained in higher yield as compared with the cases of using the trichloroacetic acid solutions according to Reference Example 1, Reference Example 2, and Reference Example 3.

### INDUSTRIAL APPLICABILITY

The present invention provides an efficient method for producing a nucleic acid oligomer. In addition, the yield of the nucleic acid oligomer to be produced in accordance with the method for producing a nucleic acid oligomer can be expected to be improved.

### [Sequence Listing Free Text]

The SEQ ID NOs: 1 to 14 of the sequence listing indicate the base sequences of oligonucleotides produced in accordance with the production method according to the present invention.

## Claims

1. A method for producing a nucleic acid oligomer represented by formula (2), the method comprising:
a step of reacting a nucleic acid oligomer represented by formula (1): 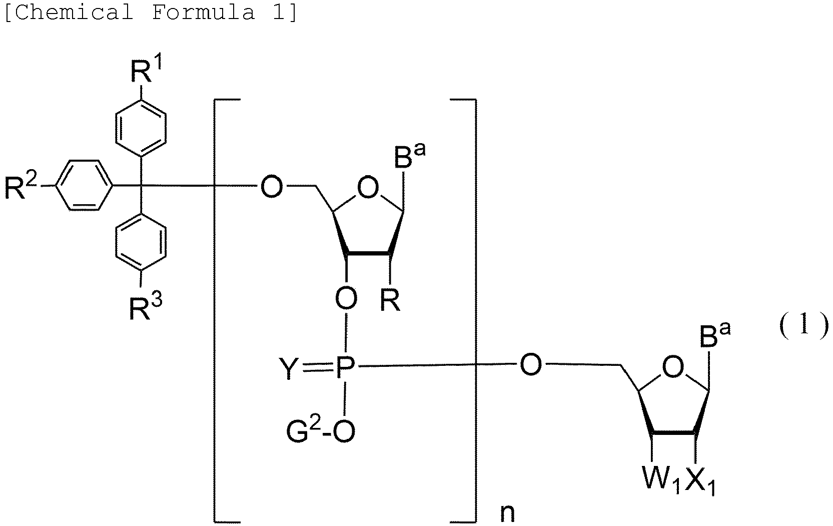 (in the formula,
G² represents a protecting group for a hydroxyl group,
B^{a} is the same or different and each independently represents a nucleobase optionally protected with a protecting group,
R¹, R², and R³ are the same or different and each independently represent a hydrogen atom or an alkoxy group,
R is the same or different and each independently represents a protected hydroxyl group, a hydrogen atom, a fluorine atom, a methoxy group, a 2-methoxyethyl group, or an OQ' group,
Q' is the same or different and each independently represents a methylene group bonded to a carbon atom at a 4'-position of a ribose, an ethylene group bonded to a carbon atom at a 4'-position of a ribose, or an ethylidene group bonded to a carbon atom at a 4'-position of a ribose,
Y is the same or different and each independently represents an oxygen atom or a sulfur atom,
n represents any integer of 1 to 200,
W₁ represents an OZ group, and X₁ represents an R group, or
W₁ represents an OV group, and X₁ represents an OZ group,
V represents a protecting group for a hydroxyl group, and
Z is a group that has a structure comprising a solid support and a linking group, and
when n is an integer of 2 or more, the nucleic acid oligomer represented by the formula (1) optionally has a non-nucleotide linker incorporated between respective nucleotides)
with a trichloroacetic acid solution in which a molar ratio of formaldehyde to trichloroacetic acid (formaldehyde mol/trichloroacetic acid mol) is 11 × 10⁻⁵ or less, 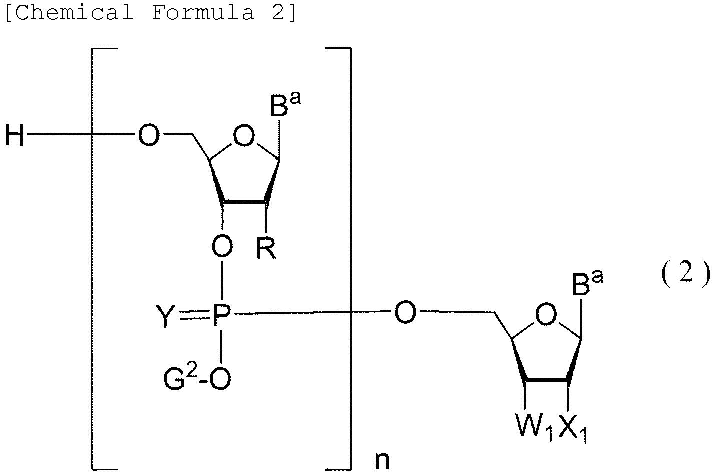 (in the formula,
G², B^{a}, R, Y, X₁, W₁, and n are as recited above, and
a non-nucleotide linker is optionally incorporated between the nucleotides as defined in the formula (1)).

2. A method for producing a nucleic acid oligomer represented by formula (2'), the method comprising:
the step according to claim 1; and further
a step of removing a group represented by Z from the nucleic acid oligomer represented by the formula (2), produced in the step according to claim 1; and
a step of removing protecting groups for a hydroxyl group and a nucleobase, 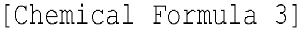 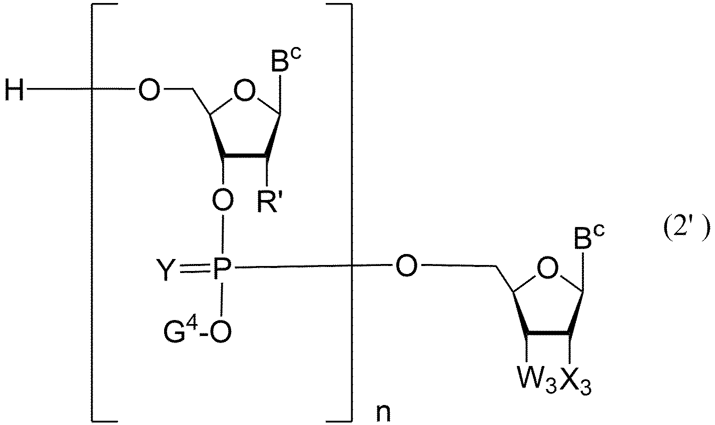 (in the formula,
Y and n are as recited above,
B^{c} is the same or different and each independently represents a nucleobase,
G⁴ is the same or different and each independently represents a hydrogen atom, an alkali metal ion, an ammonium ion, an alkylammonium ion, or a hydroxyalkylammonium ion,
R' is the same or different and each independently represents a hydroxyl group, a hydrogen atom, a fluorine atom, a methoxy group, a 2-methoxyethyl group, or an OQ' group,
Q' is as recited above, and
X₃ and W₃ each independently represent a hydroxyl group, or
X₃ represents an R' group, and W₃ represents a hydroxyl group).

3. The production method according to claim 1, further comprising:
a step of optionally elongating a chain length of the nucleic acid oligomer represented by the formula (2) by an amidite method to obtain a nucleic acid compound represented by formula (3): 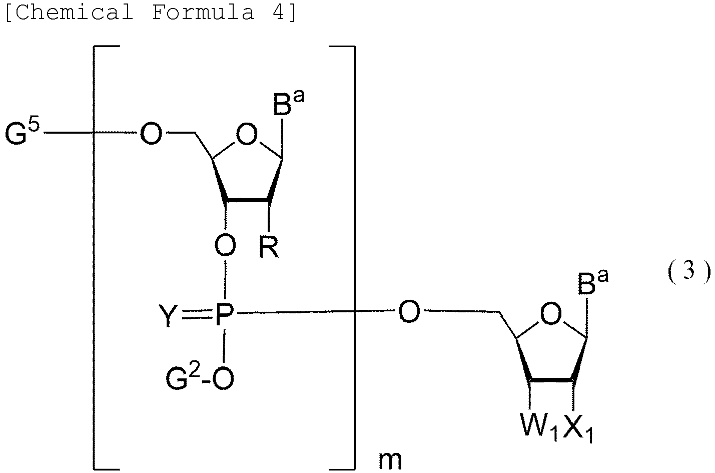 (in the formula,
G², B^{a}, R, Y, X₁, and W₁ are as recited above,
G⁵ represents a protecting group for a hydroxyl group, represented by formula: 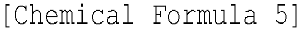 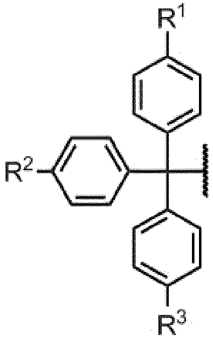
or a hydrogen atom,
R¹, R², and R³ are as recited above, and
m is an integer that satisfies m ≥ n);
a step of cutting out, from the compound represented by the formula (3), a compound represented by formula (4): 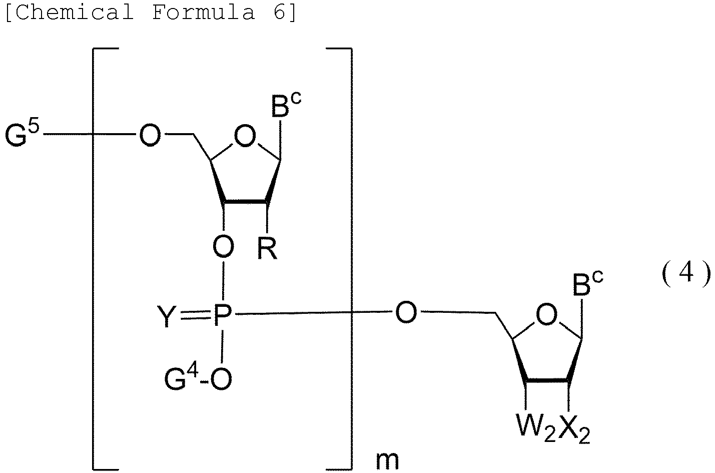 (in the formula,
G⁵, R, Y, and m are as recited above,
G⁴ is the same or different and each independently represents a hydrogen atom, an alkali metal ion, an ammonium ion, an alkylammonium ion, or a hydroxyalkylammonium ion,
B^{C} is the same or different and each independently represents a nucleobase,
X₂ represents a hydroxyl group, and W₂ represents an OV group, or
X₂ represents an R group, and W₂ represents a hydroxyl group, and
V represents a protecting group for a hydroxyl group); and further
deprotecting the compound represented by the formula (4) to produce a nucleic acid oligomer represented by formula (5): 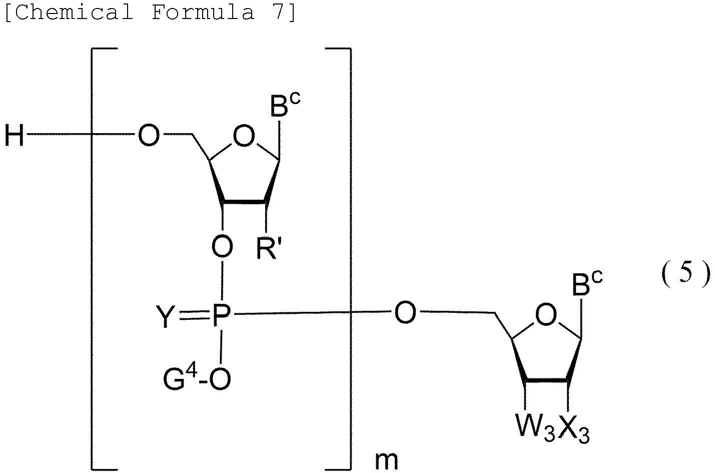 (in the formula,
G⁴, B^{c}, Y, and m are as recited above, and R' is the same or different and each independently represents a hydroxyl group, a hydrogen atom, a fluorine atom, a methoxy group, a 2-methoxyethyl group, or an OQ' group,
Q' is as recited above, and
X₃ and W₃ each independently represent a hydroxyl group, or
X₃ represents an R' group, and W₃ represents a hydroxyl group).

4. The production method according to any one of claims 1 to 3, wherein the non-nucleotide linker is a linker comprising an amino acid skeleton.

5. The production method according to claim 4, wherein the linker comprising the amino acid skeleton is a linker that has a structure selected from the group consisting of formulas (A14-1), (A14-2), and (A14-3) below: 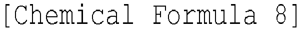 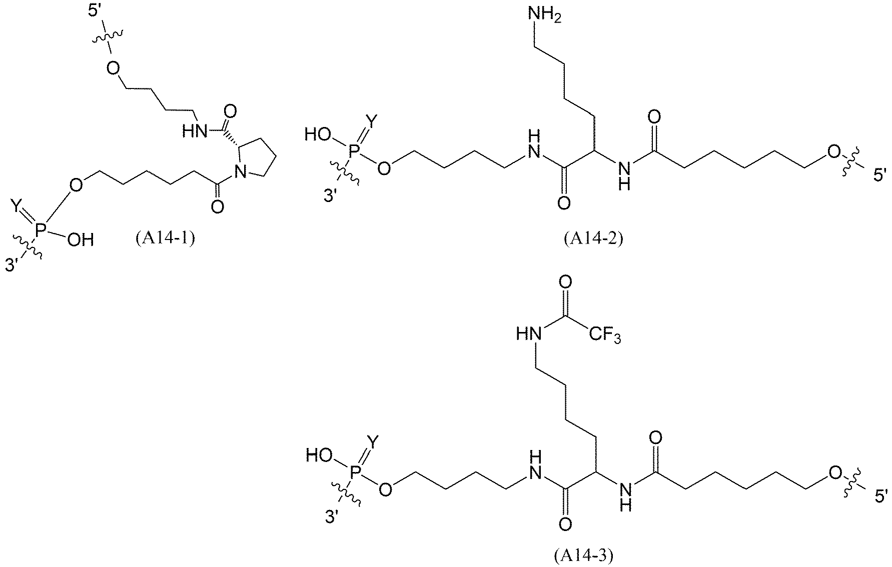 (in the formula, Y is as recited above).

6. The production method according to any one of claims 1 to 5, wherein the trichloroacetic acid solution comprises at least one solvent selected from the group consisting of dichloromethane, acetonitrile, and an aromatic organic solvent.

7. The production method according to any one of claims 1 to 6, wherein the molar ratio of the formaldehyde to the trichloroacetic acid in the trichloroacetic acid solution (formaldehyde mol/trichloroacetic acid mol) is 54 × 10⁻⁶ or less.

8. The production method according to any one of claims 1 to 6, wherein the molar ratio of the formaldehyde to the trichloroacetic acid in the trichloroacetic acid solution (formaldehyde mol/trichloroacetic acid mol) is 27 × 10⁻⁶ or less.

9. The production method according to any one of claims 1 to 8, wherein the nucleic acid oligomer is ribonucleic acid (RNA).

10. The production method according to any one of claims 1 to 8, wherein the nucleic acid oligomer is ribonucleic acid (RNA), and a protecting group for a hydroxyl group at a 2'-position of a ribose of the ribonucleic acid is a protecting group represented by formula (6), 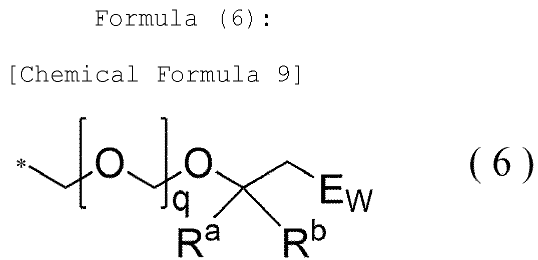 (in the formula,
q represents any integer of 1 to 5,
R^{a} and R^{b} are the same or different and each independently represent a methyl group, an ethyl group, or a hydrogen atom,
mark * represents a site bonded to an oxygen atom derived from the hydroxyl group at the 2'-position of the ribose, and
E_{W} represents an electron-withdrawing group).

11. The production method according to claim 10, wherein R^{a} and R^{b} are simultaneously hydrogen atoms, and E_{W} is a cyano group.

12. The production method according to any one of claims 1 to 11, wherein the nucleic acid oligomer is an oligomer with a chain length of 40 or more.

13. The production method according to any one of claims 1 to 11, wherein the nucleic acid oligomer is an oligomer with a chain length of 50 or more.

14. The production method according to any one of claims 1 to 11, wherein the nucleic acid oligomer is an oligomer with a chain length of 60 or more.

15. The production method according to any one of claims 1 to 11, wherein the nucleic acid oligomer is an oligomer with a chain length of 80 or more.

16. The production method according to any one of claims 1 to 11, wherein the nucleic acid oligomer is an oligomer with a chain length of 100 or more.

17. A trichloroacetic acid solution, wherein a molar ratio of formaldehyde to trichloroacetic acid (formaldehyde mol/trichloroacetic acid mol) is 11 × 10⁻⁵ or less.

18. The trichloroacetic acid solution according to claim 17, wherein the molar ratio of the formaldehyde to the trichloroacetic acid (formaldehyde mol/trichloroacetic acid mol) is 54 × 10⁻⁶ or less.

19. The trichloroacetic acid solution according to claim 17, wherein the molar ratio of the formaldehyde to the trichloroacetic acid (formaldehyde mol/trichloroacetic acid mol) is 27 × 10⁻⁶ or less.

20. A method for producing the trichloroacetic acid according to any one of claims 17 to 19, the method comprising a step of obtaining a purified trichloroacetic acid by azeotropically distilling away formaldehyde from a solution comprising: an unpurified trichloroacetic acid containing the formaldehyde; and a solvent for azeotropically distilling the formaldehyde.

21. The production method according to claim 20, wherein the azeotropic solvent has a boiling point of 198°C or lower.

22. The production method according to claim 20 or 21, wherein the azeotropic solvent is dichloromethane, acetonitrile, or an aromatic organic solvent.

23. The production method according to claim 22, wherein the aromatic organic solvent is a toluene.

24. A method for producing a nucleic acid oligomer, the method comprising: a step of purifying the trichloroacetic acid according to claim 20; and the step according to any one of claims 1 to 3, wherein the purified trichloroacetic acid obtained in the purifying step is used.

25. The production method according to any one of claims 1 to 16 and 20 to 24, comprising a step of selecting, as a trichloroacetic acid solution, the trichloroacetic acid solution according to any one of claims 17, 18, and 19.
